# EUROPEAN PATENT APPLICATION

(11) **EP 2 409 697 A1**
(43) Date of publication of application: **25.01.2012**
(21) Application number: 10170125.8
(22) Date of filing: 20.07.2010
(51) Int. Cl.: A61K 31/28, A61P 35/00

(54) **Medicaments Based on Dinuclear Ruthenium, Osmium and Iron Complexes Comprising Triply Bridging Thiolato, Selenolato, Alkoxo and/or Amido Ligands**

(71) Applicant: Université de Neuchâtel, 2001 Neuchâtel (CH)
(72) Inventor: Süss-Fink, Georg, 2000, Neuchâtel (CH); Therrien, Bruno, 2000, Neuchâtel (CH); Gras, Michael, 2000, Neuchâtel (CH); Dyson, Paul Joseph, 1024, Ecublens (CH); Zava, Olivier, 2000, Neuchâtel (CH)
(74) Representative: Schneiter, Sorin

(57) **Abstract**

The present invention provides new complexes and medicaments based on dinuclear ruthenium, osmium and iron complexes comprising triply bridging thiolato, selenolato, alkoxo and/or amido ligands. The complexes preferably comprise a M₂X₃ core, in which M is a metal atom selected from Ru, Os and Fe and each one of said three X is independently selected from said bridging ligands, with the proviso that at least one ligand comprises a substituted or unsubstituted phenyl moiety.

## Description

### Technical Field

The present invention relates to complexes for use as medicaments. The complexes are based on dinuclear ruthenium, osmium and iron complexes comprising triply bridging thiolato, selenolato alkoxo and/or amido ligands. The complexes, which are preferably provided as cations along with a pharmaceutically acceptable anionic species, are particularly useful in the treatment of hyperproliferative disorders, in particular cancer.

### Prior Art and the Problem Underlying the Invention

Cancer is the second leading cause of death in economically developed countries and the third leading cause of death in developing countries. Statistically, one in eight Europeans will develop cancer during his or her lifetime. Although estimated incidence rates show rising trends for both sexes, the age-standardized cancer mortality rate has been falling continuously among women since 1970 and among men since 1985. The increase of the survival rates is due to better cancer treatment, in particular thanks to the introduction of efficient anticancer drugs which largely contributed to this improvement.

Platinum-based drugs have been in clinical use for cancer treatment for more than 30 years. The landmark discovery of the antitumoural properties of cis-diamminedichloroplatinum(II) (cisplatin) by Rosenberg in 1965 heralded a new area of anticancer research based on metallopharmaceuticals. To date, cisplatin and its analogues are some of the most effective chemotherapeutic agents in clinical use. However, platinum-based drugs are not without problems: their high toxicity and incidence of drug resistance remain the main challenges in their clinical application.

In the search of anticancer agents containing metals other than platinum (thus overturning the platinum paradigm), ruthenium compounds turned so far out to be the most promising ones, but high activity of other metal compounds should not be excluded at the present stage.

A detailed review on the use of arene ruthenium complexes as anticancer agents was recently published by G. Süss-Fink, Dalton Trans., 2010, 39, 1673-1688.

In WO 02/40494, ruthenium-based complexes containing a 1,3,5-triaza-7-phosphaadamantane ligand were shown to induce cell death by apoptosis of SK-N-SH neuroblastoma cells, derived from human metastases at concentrations of the sub micromolar range.

In WO 2007/128158, inhibitors of resistance pathways, such as ethacryinic acid, were coupled to ruthenium-based complexes and were shown to be particularly useful in the prevention of metastasis.

WO 2009/013724 discloses high toxicity of trinuclear arene ruthenium clusters on A2780 ovarian cancer cell lines.

Mendoza-Ferri et al., Organometallics 2008, 27, 2405-2407 found high cytotoxic effects of water-soluble dinuclear arene ruthenium complexes. In this study, the length of a spacer linking two pyridinone ligand moieties was shown to have an important impact on the toxicity, with higher spacer length resulting in higher toxicity.

Very few, if any, of the compounds and complexes of the prior art cited above have resulted in clinical phase studies, not to mention actual therapies. The reasons for the poor performance of these principles are manifold and may be linked to toxicity problems or unsufficient efficiency in treatment.

It is thus an objective of the present invention to explore new ways for treating cancer, for example based on work done in the area of complexes of transition metals.

It is a further aspect of the present invention to increase the activity and/or efficiency of therapies against diseases and in particular cancer.

Considering the incidence of resistance against current therapies, it is also an objective to find new principles exhibiting toxicity against cancer cells, possibly toxic against cells that are resistant to existing therapies.

It is also an objective to provide active principles exhibiting fewer side effects than therapies of the prior art.

It is an objective to provide compounds which are still more cytotoxic against cancer cells.

It is a particular objective to provide further ruthenium-based organometallic compounds that are at least as effective as or even more effective than the organometallic compounds known so far.

The present invention addresses the problems depicted above.

### Summary of the Invention

Remarkably, the inventors provide dinuclear arene ruthenium, osmium and iron complexes comprising triply bridging thiolato, selenolato, alkoxo and/or amido ligands and demonstrate surprisingly high toxicity of such complexes against cancer cells.

The complexes preferably comprise a M₂X₃ core, in which M is a metal atom selected from Ru, Os and Fe and each one of said three X is independently selected from said bridging ligands, with the proviso that at least one ligand comprises a substituted or unsubstituted phenyl moiety. In addition, a substituted or unsubstituted arene is preferably bound as a η-6 ligand to each metal atom.

In an aspect, the present invention provides a complex of, or comprising, the structure of formula (Ia): 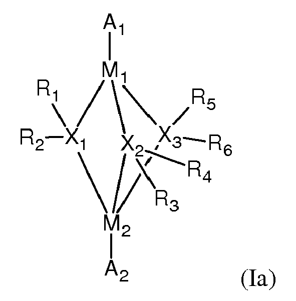
wherein M₁ and M₂ are metal atoms selected independently from Ru, Os and Fe;
wherein A₁ and A₂ are arenes bound as η-6 ligands to the metal atoms M₁ and M₂, respectively, said arenes being selected from substituted or unsubstituted benzene and substituted or unsubstituted polycycles of up to three rings and comprising at least one benzene ring;
   wherein said substituents, if present, may be selected from C1-C20 hydrocarbons optionally comprising 0-15 heteroatoms, halogens, hydroxyl, carboxylic acid group, carbonyl, nitro and amino;
wherein X₁, X₂, and X₃ are, independently, selected from S, Se, O, and N;
wherein, if any one of X₁, X₂, and X₃ is other than N, R₂, R₄, and R₆, respectively, is/are absent;
wherein R₁, R₂, R₃, R₄, R₅ and R₆, in as far as present, are selected, independently, from H, and from C1-C30 hydrocarbons optionally comprising 0-20 heteroatoms.

According to an embodiment, said C1-C30 hydrocarbons optionally comprising 0-20 heteroatoms, with respect to R₁-R₆, in as far as present, are selected from:
substituted or unsubstituted aryls;
substituted or unsubstituted aliphatic substituents; and
substituents derived independently from a natural compound and/or a bioactive compound; wherein substituents of said aryl and aliphatic substituent, if present, may be selected independently from C1-C20 hydrocarbons comprising 0-15 heteroatoms, halogens, hydroxy, carboxylic acid group, nitro and amino, and wherein two substituents provided on different carbons of said aryl or aliphatic substituent may be connected so as to form one or more ring fused to said aryl or aliphatic substituent.

According to an aspect of the invention, the complex is a cationic complex and is preferably provided together with a pharmaceutically acceptable anionic species.

In an aspect, the present invention provides complexes of the invention for use as a medicament, and/or for use in a therapeutic and/or prophylactic method.

In an aspect, the invention provides pharmaceutical composition comprising the complex of the present invention.

In an aspect, the present invention provides complexes of the invention for use in the treatment of hyperproliferative disorders, in particular cancer.

In an aspect, the present invention provides the use of the complexes of the invention in the preparation of a medicament against hyperproliferative disorders, in particular cancer.

In an aspect, the present invention provides a method for the treatment. Preferably, a condition is treated, such as a hyperproliferative disorder, in particular cancer. The method preferably comprises the step of administering, to a person in need thereof, the complex of the present invention.

Further features and advantages of the invention will become apparent to the skilled person from the embodiments described in further detail below.

### Brief Description of the Drawings

**Figure 1** shows two reaction schemes illustrating the synthesis of diene precursors used for the preparation of complexes according to the invention.
**Figure 2** shows reaction schemes illustrating the synthesis of functionalised arene ruthenium chloro intermediates **5 - 8,** which are used in the preparation of complexes according to the invention.
**Figure 3** shows reaction schemes illustrating the synthesis of functionalised ruthenium thiophenolato complexes **9 -12** encompassed by the present invention.
**Figure 4** shows reaction schemes illustrating the synthesis of the functionalised arene ruthenium hydroxythiophenolato complexes **13 -16** encompassed by the present invention.
**Figure 5** shows a reaction scheme illustrating the synthesis of unfunctionalised cationic diruthenium complex **17,** a complex according to a preferred embodiment of the present invention.
**Figure 6** shows diruthenium complexes **19** (left) and **20** (right), which are cationic complexes according to preferred embodiments of the present invention.
**Figure 7** shows the molecular structure of cation **17,** a complex according to an embodiment of the invention. Thermal ellipsoids are drawn at 50% probability level and hydrogen atoms are omitted for clarity.

### Detailed Description of the Preferred Embodiments

The present invention provides novel complexes and complexes for use in prophylactic and therapeutic applications.

According to an embodiment, the complex of the invention is of, or comprises, a structure of formula (I): 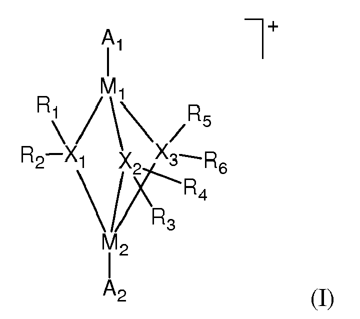
wherein M₁ and M₂ are metal atoms selected independently from Ru, Os and Fe;
wherein A₁ and A₂ are arenes bound as η-6 ligands to the metal atoms M₁ and M₂, respectively, said arenes being selected from substituted or unsubstituted benzene and substituted or unsubstituted polycycles of up to three rings and comprising at least one benzene ring;
   wherein said substituents, if present, may be selected from C1-C20 hydrocarbons optionally comprising 0-15 heteroatoms, halogens, hydroxyl, carboxylic acid group, carbonyl, nitro and amino;
wherein X₁, X₂, and X₃ are, independently, selected from S, Se, O, and N;
wherein, if any one of X₁, X₂, and X₃ is other than N, R₂, R₄, and R₆, respectively, is/are absent;
wherein R₁, R₂, R₃, R₄, R₅ and R₆, in as far as present, are selected, independently, from H, and from C1-C30 hydrocarbons optionally comprising 0-20 heteroatoms, said hydrocarbons being selected from:
   substituted or unsubstituted aryls;
   substituted or unsubstituted aliphatic substituents; and
   substituents derived independently from a natural compound and/or a bioactive compound;
wherein substituents of said aryl and aliphatic substituent, if present, may be selected independently from C1-C20 hydrocarbons comprising 0-15 heteroatoms, halogens, hydroxy, carboxylic acid group, nitro and amino, and wherein two substituents provided on different carbons of said aryl or aliphatic substituent may be connected so as to form one or more ring fused to said aryl or aliphatic substituent.

For the purpose of the present specification, the term "comprise", or "comprising" is intended to mean "includes, amongst others" and is not intended to mean "consists only of".

In the terms of the present specification and the appended claims, the expression "selected from", may, according to an embodiment, be interpreted as "selected from the group consisting of", if applicable in the context, in particular if followed by a specific list of options.

"Heteroatoms", for the purpose of the present specification, may be selected from any atom other than carbon and hydrogen. According to an embodiment, metal atoms are excluded, preferably, alkali metals are excluded, even more preferably, transition metals are also excluded. According to a preferred embodiment, heteroatoms are selected from non-metals, in particular from B, N, P, As, O, S, Se, and from halogen, halogen encompassing preferably F, Cl, Br, and I. According to a preferred embodiment, heteroatoms are selected from N, O, S, and halogen. Heteroatoms other than halogen may, if applicable, replace a carbon atom within a hydrocarbon molecule, for example in a carbon chain or in a ring. Such heteroatoms are preferably selected from N, O and S.

According to a preferred embodiment, M₁ and M₂ are both the same metal. Preferably, M₁ and/or M₂ are Ru.

X₁, X₂, and X₃ may be selected independently from S, Se, O, and N.

According to a preferred embodiment, X₁, X₂, and X₃ are all the same.

Preferably, X₁, X₂, and X₃ are selected independently from S, Se, O, more preferably from S and Se. Most preferably X₁, X₂, X₃ all are sulfur atoms.

If any one, any two or all three of X₁, X₂, and X₃ are N, the respective X may have two separate single bonds to H or other substituents, in particular R₁ and R₂ on X₁, R₃ and R₄ on X₂ and R₅ and R₆ on X₃.

If any one, any two or all three of X₁, X₂, and X₃ are other than N, the respective X has only one single bond to H or other substituents, in particular R₁ on X₁, R₃ on X₂ and R₅ on X₃, the respective other substituent shown in the complex of formulae (I) and (Ia) being absent (any one, any two or all three of: R₂ on X₁, R₄ on X₂ and R₆ on X₃ are absent, respectively, in this case).

According to an embodiment, at least one, preferably at least two, and more preferably at least three of substituents R₁, R₂, R₃, R₄, R₅ and R₆, in as far as present, are different from H.

According to an embodiment, any one of R₁, R₂, R₃, R₄, R₅ and R₆ is different from H, unless if bound to a nitrogen atom (X₁, X₂, and/or X₃, respectively, being N), and if the neighboring substituent bound to the same nitrogen atom is different from H. For example if X₁ is N, one of R₁ and R₂ is H and the respective other is different from H; if X₂ is N, one of R₃ and R₄ is H and the respective other is different from H, and/or, independently, if X₃ is N, one of R₅ and R₆ is H and the respective other is different from H.

In the complex of the invention, R₁, R₂, R₃, R₄, R₅ and R₆, in as far as present, are selected, independently, from H, and from C1-C30 hydrocarbons optionally comprising 0-20 heteroatoms, as further defined in this specification. Preferably, said hydrocarbon is a C2-C20 hydrocarbon comprising 0-10 heteroatoms, more preferably a C3-C15 hydrocarbon comprising 0-8 heteroatoms and most preferably a C6-C10 hydrocarbon comprising from 0-3 heteroatoms.

In accordance with an embodiment of the invention, R₁, R₂, R₃, R₄, R₅ and R₆, in as far as present, may be selected from substituted or unsubstituted aryls. Aryls may be selected from heteroaryls or aryls lacking heteroatoms. Aryls are preferably selected from C6-C12 aryls and more preferably from phenyl and naphtyl. Preferred substituents of said aryl are as defined elsewhere in this specification and may be selected, independently, from the same substituents as those of said aliphatic substituents.

In accordance with an embodiment of the invention, R₁, R₂, R₃, R₄, R₅ and R₆, in as far as present, may be selected from substituted or unsubstituted aliphatic substituents. Said aliphatic substituent is preferably selected from C1-C10 alkyl, C2-C10 alkenyl, C2-C10 alkynyl, wherein said alkyl, alkenyl and alkynyl, if it comprises at least three carbons, may be linear, branched and/or cyclic and may be substituted, and wherein, in said alkyl, alkenyl and alkynyl, one or more carbons may be replaced by heteroatoms. Although this is encompassed by the invention, preferably, however, in said alkyl, alkenyl, and alkynyl, no carbon is replaced by a heteroatom. It is noted that substituents of said aliphatic substituent may not be aliphatic but aromatic. Preferred substituents of said aliphatic substituents are as defined elsewhere in this specification and may be the same as for said aryl substituents.

In R₁-R₆, in as far as present, substituents of said aryl and aliphatic substituents, if present, may be selected, independently, from C1-C20 hydrocarbons optionally comprising 0-15 heteroatoms, preferably C1-C15 hydrocarbons optionally comprising 0-10 heteroatoms, more preferably C1-C10 hydrocarbons comprising 0-5 heteroatoms, even more preferably C1-C5 hydrocarbons comprising 0-3 heteroatoms.

According to a preferred embodiment, in said R₁, R₂, R₃, R₄, R₅ and R₆, in as far as present, said aryl is a substituted or unsubstituted phenyl; said aliphatic substituent is selected from C1-C10 alkyl, C2-C10 alkenyl, C2-C10 alkynyl, wherein said alkyl, alkenyl and alkynyl may be further substituted and may be linear and, if it comprises at least three carbons, also branched and/or cyclic and, and wherein, in said alkyl, alkenyl ad alkynyl, one or more carbons may be replaced by heteroatoms; wherein substituents of said phenyl, alkyl, alkenyl, and alkynyl, if present, may be independently selected from: C1-C20 hydrocarbons optionally comprising 0-15 heteroatoms, or preferred hydrocarbon substituents as mentioned above.

In an embodiment, if R₁, R₂, R₃, R₄, R₅ and R₆ is an aryl or a cyclic aliphatic substituent (such as a cyclic alkyl, alkenyl or alkynyl, for example, as defined above), substituents of said aryl and cyclic aliphatic substituent may be connected to each other to form a ring, which may be, for example, fused to said aryl or cyclic aliphatic substituent.

According to a preferred embodiment, with respect to R₁-R₆, in as far as present, said optional substituents of said aryl (preferably: phenyl), alkyl, alkenyl, and alkynyl, if present, are selected from: halogen; -R₃₀; -O-R₃₀; -O-R₃₅; wherein R₃₀ is independently selected from C1-C8 alkyls, C2-C8 alkenyls, and C2-C8 alkynyls, which, independently, may be linear, and if comprising more than three carbons, branched or cyclic, and which may be totally or partially halogenated, and from substituents of formula (V) below: 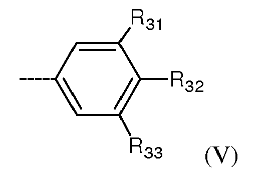 wherein R₃₅ is derived from a C3-C20 hydrocarbon compound comprising 1 to 15 heteroatoms, said compound being selected from natural compounds and bioactive compounds comprising at least one carboxylic acid group forming, in said substituent R₃₅, an ester bond with the oxygen atom of said -O-R₃₅ substituent; wherein R₃₁, R₃₂, R₃₃ are, independently, selected from: H; halogen; C1-C8 alkyl, C2-C8 alkenyl, C2-C8 alkynyl, which may linear or, if comprising three or more carbons, branched and cyclic, wherein said alkyls, alkenyls and alkynyl may be totally or partially halogenated; and, substituents -O-R₃₅. According to an embodiment, said C1-C8 alkyl, C2-C8 alkenyl, C2-C8 alkynyl are selected from C1-C4 alkyls, C2-C4 alkenyls and C2-C4 alkynyls, respectively.

According to an embodiment, R₃₅ is derived from any one compound selected from isonicotinic acid, ethacrynic acid, folic acid, biotin, pyridoxol-5-phosphate hydrate, wherein any carboxylic acid group of said compound forms an ester bond with the oxygen atom of the -O-R₃₅ substituent.

In accordance with the invention and embodiments thereof, wherein R₁, R₂, R₃, R₄, R₅ and R₆, in as far as present, may be, independently, a substituent derived from a natural compound and/or a bioactive compound.

According to an embodiment, if said substituents R₁, R₂, R₃, R₄, R₅ and/or R₆, in as far as present, is derived from a natural compound and/or bioactive compound, said natural and/or bioactive compound, when not yet bound in the complex of the invention, comprises at least one group selected from HS-, HSe-, HO-, a primary amino group, a secondary amino group (said group being, for the purpose of the present specification and with respect to substituents R₁-R₆, a "ligand group", as it forms said thiolato, selenolato, alkyxo or amido ligand in the complex of the invention), or wherein said natural or bioactive compound is covalently connected to a linker moiety comprising one of the aforementioned ligand groups, the linker moiety comprising, preferably consisting of, said ligand group included, 1-10 carbons and 1-5 heteroatoms, and wherein, in said substituent derived from a natural compound and/or a bioactive compound, the sulphur, selenium, oxygen or nitrogen atom of said HS-, HSe-, HO-, a primary amino group, a secondary amino group, respectively, constitutes one or more of X₁, X₂, and X₃ in the complex. In other words, the S, Se, O or N atom of said HS-, HSe-, HO-, primary amino, and/or secondary amino ligand group, respectively, of said natural or bioactive compound or of said linker forms said thiolato, selenolato, alkyxo or amido ligand, with said H of said ligand group being removed.

Preferably, said linker moiety comprises, said group included, 1-5 carbons and 1-4 heteroatoms, more preferably 1-3 carbons and 1-2 heteroatoms.

Examples of natural compounds for the purpose of this specification (also with respect to -OR₃₅) are naturally occurring carbohydrates; such as mono-, di-, and polysaccharides; amino acids and peptides, such as di-, tri-, oligo-, and polypeptides; lipids, such as fatty acids, phospholipids, ceramides and other sphingolipids, such as aphingoglycolipids; terpenes, in particular sesqui-, mono-, di-, sester-, tri-, tetra-, and polyterpenes; alkaloids; lipoproteins; glycolipids, such as glyceroglycolipids; glycoproteins, just to mention a few.

Specific natural and bioactive compounds are as listed further above with respect to -R₃₅.

For example, a carboxylic acid group of such a natural or bioactive compound may be esterified with a hydroxyl group provided on the linker, and the ligand group of said linker thus providing said bivalent thiolato, selenolato, alkoxo and/or amido ligand to metal M₁ and M₂ with substituent R₁-R₆, as applicable.

According to a preferred embodiment, said bioactive moiety or said natural or bioactive compound is a compound comprising a thiol or a selenol group. When said bioactive or natural compound forms a hydrocarbon substituent in the compound of the invention (X₁-R₁, X₂-R₃, and/or X₃-R₅), the sulphur or selenium atom of said thiol or selenol group is the sulphur or selenium atom of the thiolato or selenato ligand, respectively, by which the bioactive or natural compound is bound to the metal M₁ and M₂.

Examples of natural or bioactive compounds comprising free thiol or selenol groups and which could be used as thiolato or selenolato ligand (S-R₁₋₆; Se-R₁₋₆), for example, may be selected from cysteine, selenocysteine, peptides comprising a cysteine or selenocysteine moiety, thioglucose, for example.

According to a preferred embodiment, at least one, preferably at least two, most preferably at least three of R₁, R₂, R₃, R₄, R₅ and R₆, in as far as present, are selected independently from substituted or unsubstituted aryl, preferably phenyl, substituents being as defined elsewhere in this specification and preferably selected from C1-C4 alkyl, which may be linear or, if there are three or more carbons, may be branched and/or cyclic.

According to a preferred embodiment, X₁, X₂ and X₃ are selected, independently, from S, Se and O, and at least one, preferably at least two, and, according to an embodiment, all three of R₁, R₃ and R₅ are selected independently from substituted and unsubstituted aryl, in particular phenyl, wherein substituents of said phenyl, may be selected, independently as defined elsewhere in this specification, in particular from phenyl, and C1-C4 alkyl.

According to an embodiment, one, two, three or more substituents of R₁, R₂, R₃, R₄, R₅ and R₆, in as far as present, are H or hydrocarbons as defined elsewhere in this specification, with the proviso that they are free of any heteroatom and/or that definitions of substituents that do not contain any heteroatom are encompassed, independently from the other substituents R₁-R₆.

In particular, said optionally substituted aryl or aliphatic substituents are preferably free of heteroatoms, whereas substituents thereof may contain heteroatoms, but are preferably also free of heteroatoms.

According to an embodiment, one, two, three or more of R₁, R₂, R₃, R₄, R₅ and R₆, in as far as present, are selected from H and hydrophobic substituents.

As mentioned above, A₁ and A₂ in formula (I) above are arenes bound as η-6 ligands to the metal atoms M₁ and M₂, respectively, said arenes being selected from substituted or unsubstituted benzene and substituted or unsubstituted polycycles of up to three rings and comprising at least one benzene ring. Substituents of A₁ and A₂, if present, may be selected from C1-C20 hydrocarbons optionally comprising 0-15 heteroatoms, halogens, hydroxyl, carboxylic acid group, carbonyl, nitro and amino. The carboxylic acid group may be further substituted, for example alkylated.

A₁ and A₂ may be the same or may be different. According to a preferred embodiment, A₁ and A₂ are the same, that is, are substantially identical.

It is noted that the substituents of the arene A₁ and A₂ do not seem to have a huge impact on the activity of the complexes, which is why substituents may be selected from a large group of substituents. It is thus not excluded to provide additional functionalities on the arene ring, such as, for example, disclosed in W02007/128158, in particular on page 9, line 25 to page 11, line 21 of the published international application. There, the possibility of a bioactive compound bound to the arene is also encompassed. W02007/128158 is entirely incorporated herein by reference.

According to a preferred embodiment, A₁ and A₂ are, independently, selected from arenes of formula (VI) below: 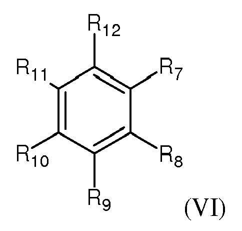 wherein R₇, R₈, R₉, R₁₀, R₁₁, and R₁₂, are independently selected from H and C1-C20 hydrocarbons optionally comprising 0-15 heteroatoms, halogens, hydroxyl, carboxylic acid group, carbonyl, nitro and amino. The carboxylic acid group may be further substituted, for example alkylated. Different substituents may be may be linked so as to form one or more rings fused to said benzene ring. Preferably, substituents of neighbouring carbons of the benzene rings may be linked so as to for a ring fused to said benzene ring. The ring fused to the benzene may form a polycyclic arene, such as naphthalene, inden, anthracene phenantrene, biphenylene, and the like, for example.

According to an embodiment, A₁ and A₂ are, independently, selected from arenes of formula (VI), (VII), (VIII), (IX), (X), (XI) and (XII) below: 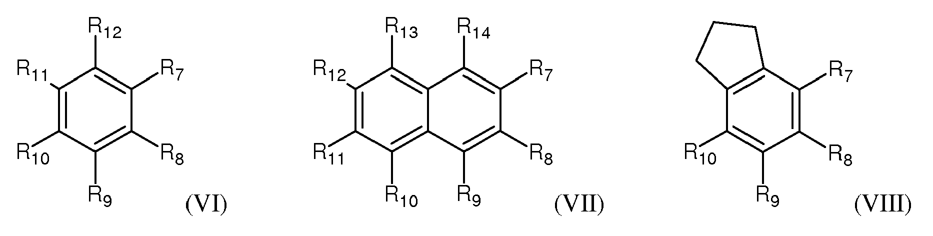 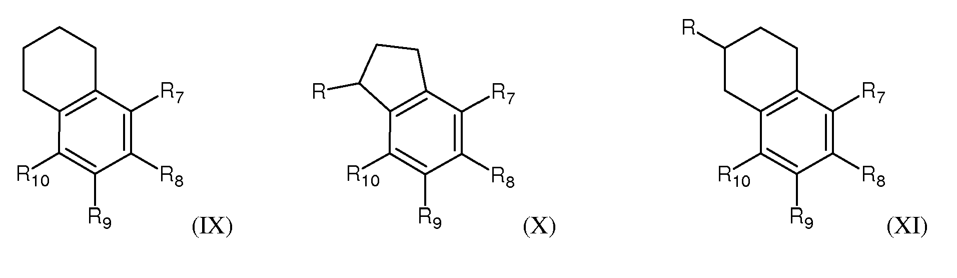 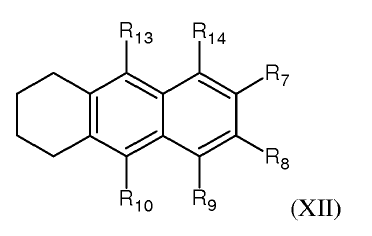 wherein R, R₇, R₈, R₉, R₁₀, R₁₁, R₁₂, R₁₃, R₁₄, are as R₇ to R₁₂, defined above.

The arenes of formula (VIII) to (XII), are examples in which substituents R₁₁ and R₁₂ are linked so as to form a ring fused to the benzene ring. Arene (VII) is substituted or unsubstituted naphthalene, an example for a substituted or unsubstituted polycycle. Arene (XII) is the same as (VII), in which substituents R₁₁ and R₁₂ are linked (covalently) so as to form a system comprising a ring fused to the naphthalene ring system.

Since A₁ and A₂ may be the same or may be different, and since the substituents on A₂ may be selected independently from those on A₁, it can be said that A₁may be selected of arenes of formula (VI) below and A₂ may be selected of arenes of formula (XIII) below. 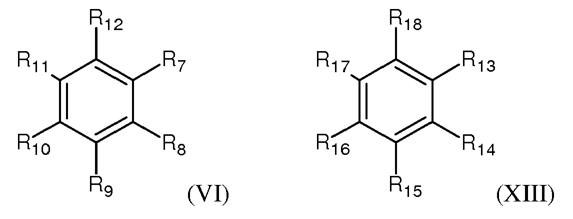 wherein R₇, R₈, R₉, R₁₀, R₁₁, R₁₂, R₁₃, R₁₄, R₁₅, R₁₆, R₁₇, and R₁₈ may be selected independently from substituents as defined for R₇ to R₁₂ with respect to formula (VI) above.

According to a preferred embodiment, R₇, R₈, R₉, R₁₀, R₁₁, R₁₂, R₁₃, R₁₄, R₁₅, R₁₆, R₁₇, and R₁₈ are independently selected from C4-C20 hydrocarbons optionally comprising 0-15 heteroatoms, with the proviso that said C4-C20 hydrocarbon optionally comprising 0-15 heteroatoms comprises at least one aromatic ring or ring system and/or comprises at least one bioactive moiety, and further from H, halogens, hydroxyl, carboxylic acid group, nitro and amino, C1-C10 alkyls, C2-C10 alkenyls, C2-C10 alkynyls, C4-C10 aryl, C1-C10 alkoxyl, C2-C10 alkenoxyl, C2-C10 alkynoxyl, C4-C10 aryloxyl, all of which may be totally or partially halogenated and wherein said alkyl, alkoxyl, alkenyl, alkenoxyl, alkynyl, alkynoxyl, may be linear or branched.

Any aryl and/or an aryl moiety of said aryloxyl mentioned herein, if it is a C4 or a C5 aryl and/or aryloxyl, respectively, it comprises at least one heteroatom. Said aryl and aryloxyl may be further substituted, besides with halogen, with C1-C4 alkyl, and wherein substituents on neighbouring carbons of the benzene ring may be linked so as to form one or more rings fused to said benzene ring.

According to a preferred embodiment, R₇, R₈, R₉, R₁₀, R₁₁, R₁₂, R₁₃, R₁₄, R₁₅, R₁₆, R₁₇, and R₁₈ are independently selected from H, halogens, hydroxyl, carboxylic acid group, nitro and amino, C1-C8 alkyls, C2-C8 alkenyls, C2-C8 alkynyls, C4-C8 aryl, C1-C8 alkoxyl, C2-C8 alkenoxyl, C2-C8 alkynoxyl, C4-C10 aryloxyl, wherein said alkyl, alkoxyl, alkenyl, alkenoxyl, alkynyl, alkynoxyl, and aryloxyl may be totally or partially halogenated and/or may be linear or branched, wherein, if said aryl and/or an aryl moiety of said aryloxyl, if it is a C4 or a C5 aryl and/or aryloxyl, respectively, it comprises at least on heteroatom, and wherein substituents on neighbouring carbons of the benzene ring may be linked so as to form one or more rings fused to said benzene ring. Said fused rings, may, of course, be further substituted, as far as other requirements as defined herein are met.

According to an embodiment, R₇, R₈, R₉, R₁₀, R₁₁, R₁₂, R₁₃, R₁₄, R₁₅, R₁₆, R₁₇, and R₁₈ are independently selected from H, C1-C5 alkyls, C2-C5 alkenyls, and C2-C5 alkynyls, wherein said alkyl, alkenyl and alkynyl may be linear or branched, and from - (CH₂)ₙ-O-C(O)-C₆H₄-*p*-O-(CH₂)ₘCH₃, -(CH₂)ₙ-O-C(O)-CH=CH-C₆H₄-*p*-O-CH₃, wherein m is, independently, an integer of 1-6 and n being, independently, 1, 2, 3 or 4.

According to an embodiment, R₇, R₈, R₉, R₁₀, R₁₁, R₁₂, R₁₃, R₁₄, R₁₅, R₁₆, R₁₇, and R₁₈ are independently selected from H, and C1-C4 alkyls.

In the most active compounds, R₇, R₈, R₉, R₁₀, R₁₁, R₁₂, R₁₃, R₁₄, R₁₅, R₁₆, R₁₇, and R₁₈ are independently selected from H and C1 to C3 alkyls, although complexes with arenes comprising one longer and more complex substituents also comprising heteroatoms as defined herein (for example, -(CH₂)ₙ-O-C(O)-C₆H₄-*p*-O-(CH₂)ₘCH₃) were also found to be highly active.

According to an embodiment, one substituent of R₇, R₈, R₉, R₁₀, R₁₁, and R₁₂, and/or, independently, one substituent of R₁₃, R₁₄, R₁₅, R₁₆, R₁₇, and R₁₈ is selected, from C4-C20 hydrocarbons optionally comprising 0-15 heteroatoms, with the proviso that said C4-C20 hydrocarbon optionally comprising 0-15 heteroatoms comprises at least one aromatic ring or ring system and/or comprises a radical or substituent derived from at least one bioactive compound, the entire substituents thus forming or containing a bioactive moiety, possibly linked by way of suitable linker. The other substituents are selected independently according to the preferred embodiments mentioned above, preferably from H and C1-C4 alkyls.

Preferred arenes, from which A₁ and A₂ may be independently selected are C₆H₆ (benzene), C₆H₅Me (methylbenzene), C₆Me₆ (hexamethylbenzene), *p*-ⁱPrC₆H₄Me (*p*-cymene), C₉H₁₀ (indane), C₆H₆-(CH₂)ₙ-O-C(O)-C₆H₄-*p*-O-(CH₂)ₘCH₃, C₆H₆-(CH₂)ₙ-O-C(O)-CH=CH-C₆H₄-*p*-O-CH₃, with m being an integer of 1-6 and n being 1, 2, 3 or 4.

According to an embodiment, the present invention provides a complex of, or comprising, a structure selected from formula (IIa) and (II) below: 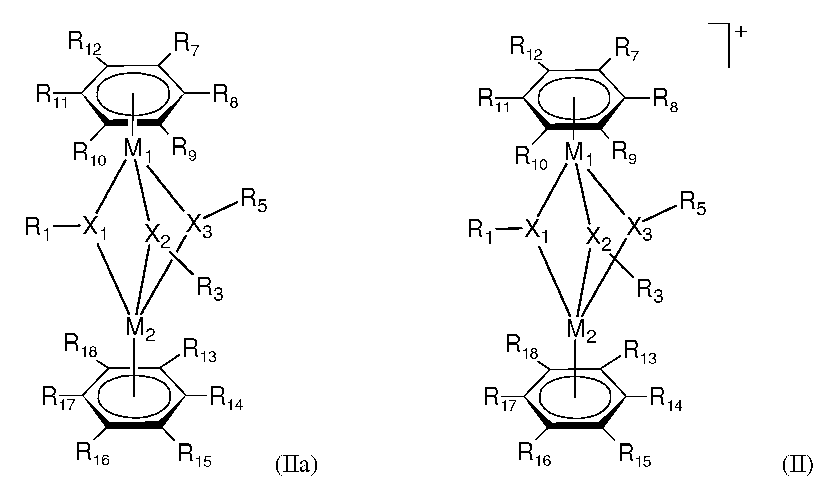
wherein M₁, M₂, R₁, R₃ and R₅, are, independently, as defined elsewhere in this specification, in particular above and below, for example such as defined with respect to any one of (I), (Ia), (II), (IIa), (III), (IIIa), (IV), (IVa), (IVb) in as far as applicable;
wherein X₁, X₂ and X₃ are selected from S, Se and O;
wherein substituents R₇, R₈, R₉, R₁₀, R₁₁, R₁₂, R₁₃, R₁₄, R₁₅, R₁₆, R₁₇, and R₁₈ are selected, independently, from: C4-C20 hydrocarbons optionally comprising 0-15 heteroatoms, with the proviso that said C4-C20 hydrocarbon optionally comprising 0-15 heteroatoms comprises at least one aromatic ring or ring system and/or comprises at least one bioactive moiety, and further from: H, halogens, hydroxyl, carboxylic acid group, nitro and amino, C1-C10 alkyls, C2-C10 alkenyls, C2-C10 alkynyls, C4-C10 aryl, C1-C10 alkoxyl, C2-C10 alkenoxyl, C2-C10 alkynoxyl, C4-C10 aryloxyl, wherein said alkyl, alkoxyl, alkenyl, alkenoxyl, alkynyl, alkynoxyl, may be totally or partially halogenated and/or may be linear or branched, wherein said aryl and/or an aryl moiety of said aryloxyl, if it is a C4 or a C5 aryl and/or aryloxyl, respectively, it comprises at least on heteroatom, wherein said aryl and aryloxyl may be further substituted, besides with halogen, with C1-C4 alkyl, and wherein substituents on neighbouring carbons of the benzene ring may be linked so as to form one or more rings fused to said benzene ring. Of course, this fused ring may be further substituents, as far as the other requirements on the respective substituents that are fused are met. Preferred embodiments and examples of M₁, M₂, R₁, R₃, R₅, X₁, X₂, X₃, R₇, R₈, R₉, R₁₀, R₁₁, R₁₂, R₁₃, R₁₄, R₁₅, R₁₆, R₁₇, and R₁₈ as appearing in formula (II) are, independently, as detailed, exemplified and defined elsewhere in this specification, in particular herein above and herein below, with respect to any complex of formula (I), (Ia), (III), and (IV) and other structures, partial structures and substituents disclosed herein.

According to an embodiment, the present invention provides a complex of or comprising a structure selected from formula (IIIa) and formula (III): 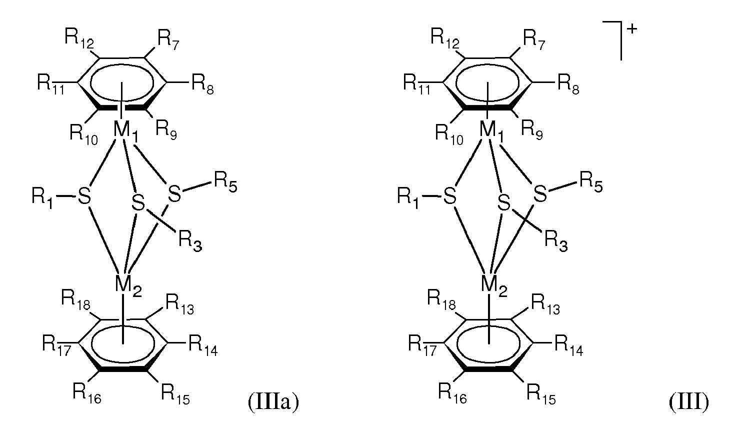 wherein:
M₁ M₂, R₁ R₃, R₅, R₇, R₈, R₉, R₁₀, R₁₁, R₁₂, R₁₃, R₁₄, R₁₅, R₁₆, R₁₇, and R₁₈ are independently as detailed, exemplified and defined elsewhere in this specification, in particular herein above and herein below, with respect to any complex of formula (I), (Ia), (II), (IIa), (IV) and (IVa), in as far as applicable, and other structures, partial structures and substituents disclosed herein.

According to an embodiment, the present invention provides a complex of or comprising a structure selected from formula (IVa) and formulae (IV): 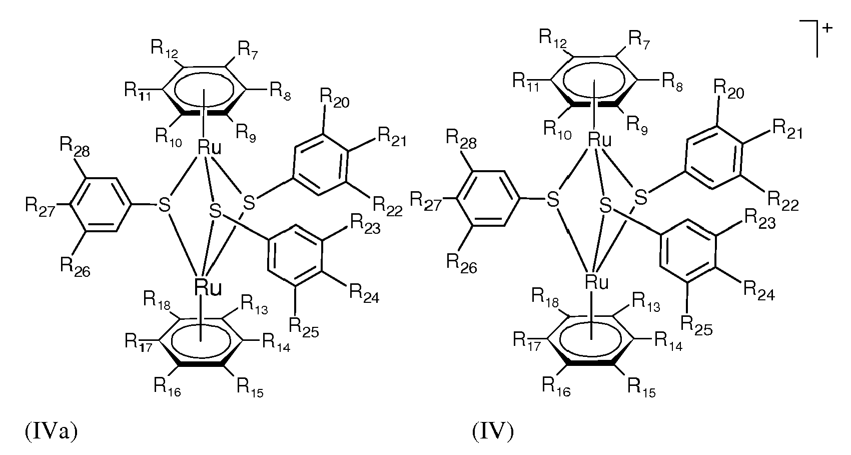 wherein:
R₇, R₈, R₉, R₁₀, R₁₁, R₁₂, R₁₃, R₁₄, R₁₅, R₁₆, R₁₇, and R₁₈ are independently as detailed, exemplified and defined elsewhere in this specification, in particular herein above and herein below, with respect to any complex of formula (I), (Ia), (II), (IIa), (III) and (IIIa) and other structures, partial structures and substituents disclosed herein, in as far as applicable. R₂₀, R₂₁, R₂₂ R₂₃, R₂₄, R₂₅, R₂₆, R₂₇, R₂₈ may independently be selected from H, halogen, -R₃₀, -OR₃₀, -O-R₃₅, wherein -R₃₀ and -O-R₃₅ are defined as disclosed elsewhere in this specification. According to an embodiment, -R₃₀ is independently selected from H and C1-C4 alkyl.

According to an embodiment, the present invention provides a complex of, or comprising, a structure of formula (IVb) below: 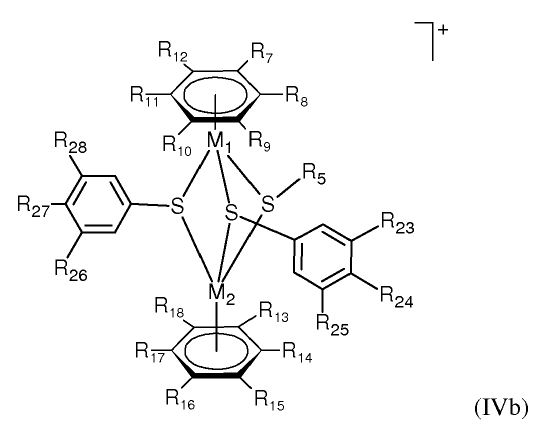 in which any one of M₁, M₂, (preferably Ru), R₅, R₇, R₈, R₉, R₁₀, R₁₁, R₁₂, R₁₃, R₁₄, R₁₅, R₁₆, R₁₇, R₁₈, R₂₃, R₂₄, R₂₅, R₂₆, R₂₇, R₂₈ are, independently, as defined elsewhere in this specification, in particular with respect to the structures of formula (I), (Ia), (II), (IIa), (III), (IlIa), (IV), (Iva), in as far as applicable. For example, R₅ is selected from H; substituted or unsubstituted aliphatic substituents; and from substituents derived independently from a natural compound and/or a bioactive compound, as disclosed hereinabove, for example with respect to R₁-R₆ of formulae (Ia) and/or (I) above.

According to an embodiment, one, or two, or all three groups of substituents located on a specific benzenethiolate ligand (e.g. S-phenyl in formula Iva or IV) (group 1: R₂₀, R₂₁, R_{22;} group 2: R₂₃, R₂₄, R₂₅; group 3: R₂₆, R₂₇, R₂₈) are selected independently as defined elsewhere in this specification, for example above or below, with the proviso that they are free of any heteroatom, and/or that definitions of substituents that do not contain any heteroatom are encompassed, independently from the other substituents.

According to an embodiment, one, or two, or all three groups of substituents located on a specific benzenethiolato ligand (phenyl) (group 1: R₂₀, R₂₁, R₂₂; group 2: R₂₃, R₂₄, R₂₅; group 3: R₂₆, R₂₇, R₂₈), in as far as present, are selected independently from H and hydrophobic substituents.

According to an embodiment of the complex of formula (IV), R₂₀, R₂₁, R₂₂, R₂₃, R₂₄, R₂₅, R₂₆, R₂₇, R₂₈, in as far as present, are selected, independently, from H, C1-C4 alkyl, wherein said alkyl, if it has 3 or 4 carbons, may be branched. Even more preferably, R₂₀, R₂₁, R₂₂, R₂₃, R₂₄, R₂₅, R₂₆, R₂₇, R₂₈, in as far as present, are selected, independently, from H and methyl.

According to a preferred embodiment, the complex of the present invention is cationic, preferably carrying one positive charge, as exemplified by the structures of formulae (I), (II), (III), and (IV).

According to an embodiment, the complex of the present invention has the formula [(arene)₂Ru₂(SY)₃]⁺, wherein arene is selected from C₆H₆ (benzene), C₆H₅Me (methylbenzene), C₆Me₆ (hexamethylbenzene), *p*-ⁱPrC₆H₄Me (*p*-cymene), C₉H₁₀ (indane), C₆H₆-(CH₂)ₙ-O-C(O)-C₆H₄-*p*-O-(CH₂)ₘCH₃, C₆H₆-(CH₂)ₙ-O-C(O)-CH=CH-C₆H₄-*p*-O-CH₃, with m being an integer of 1-6 and n being 1, 2, 3 or 4; and, Y is selected from phenyl and C1-C3 alkylphenyl, in particular *p*-C1-C3 alkylphenyl.

According to an embodiment, the complex of the present invention is or comprises at least one binuclear ruthenium cation selected from the group of:
[(C₆H₆)₂Ru₂(SPh)₃]⁺, [(C₆H₆)₂Ru₂(S-*p*-C₆H₄Me)₃]⁺, [(C₆H₅Me)₂Ru₂(S-Ph)₃]⁺,
[(C₆H₅Me)₂Ru₂(S-*p*-C₆H₄Me)₃]⁺, [(C₆Me₆)₂Ru₂(SPh)₃]⁺, [(C₆Me₆)₂Ru₂(S-*p*-C₆H₄Me)₃]⁺,
[(*p*-ⁱPrC₆H₄Me)₂Ru₂(SPh)₃]⁺, [(*p*-ⁱPrC₆H₄Me)₂Ru₂(S-*p*-C₆H₄Me)₃]⁺,
[(C₆H₆-(CH₂)₂-O-C(O)-C₆H₄-*p*-O-(CH₂)₆CH₃)₂Ru₂(SPh)₃]⁺,
[(C₆H₆-(CH₂)₂-O-C(O)-C₆H₄-*p*-O-(CH₂)₆CH₃)₂Ru₂(S-*p*-C₆H₄Me)₃]⁺,
[(C₆H₆-(CH₂)₄-O-C(O)-C₆H₄-*p*-O-(CH₂)₆CH₃)₂Ru₂(SPh)₃]⁺,
[(C₆H₆-(CH₂)₄-O-C(O)-C₆H₄-*p*-O-(CH₂)₆CH₃)₂Ru₂(S-*p*-C₆H₄Me)₃]⁺,
[*trans*- (C₆H₆-(CH₂)₂-O-C(O)-CH=CH-C₆H₄-*p*-O-CH₃)₂Ru₂(SPh)₃]⁺,
[*trans*- (C₆H₆-(CH₂)₂-O-C(O)-CH=CH-C₆H₄-*p*-O-CH₃)₂Ru₂(S-*p*-C₆H₄Me)₃]⁺,
[*trans*-(C₆H₆-(CH₂)₄-O-C(O)-CH=CH-C₆H₄-*p*-O-CH₃)₂Ru₂(SPh)₃]⁺, and
[*trans*-(C₆H₆-(CH₂)₄-O-C(O)-CH=CH-C₆H₄-*p*-O-CH₃)aRu₂(S-*p*-C₆H₄Me)₃]⁺.

According to an embodiment, the complex of the invention is provided together with a pharmaceutically acceptable anionic species. The anionic species may be a pharmaceutically acceptable anion. According to a preferred embodiment, the anion is chloride.

Alternatively, the cationic complex of the invention may be provided together with a structure of higher molecular weight, carrying a plurality of negative charges, with several cationic complexes of the invention being associated with the supramolecular structure. For example, the cationic complex may be provided with one or more polyanions, for example anionic polyelectrolytes, for example artificial or natural polymers comprising a plurality of negative charges, such as anionic polypeptides, polysaccharides and the like, for example.

The invention also encompasses nanoparticles comprising the complex of the invention, in particular magnetic and/or ferromagnetic nanoparticles with modified surfaces so as to carry negative charges at the surface. Such nanoparticles may be helpful in targeting the complexes of the invention to specific cells.

The complexes of the invention exhibit high cytotoxicity against cancer cells. The complexes may thus be used as medicaments, in particular for the treatment of hyperproliferative disorders, in particular cancer.

According to an embodiment, the complexes of the invention are used in the treatment of any one or more selected independently from the group of a carcinoma, including a carcinoma of the bladder, breast, colon (e.g. colorectal carcinomas such as colon adenocarcinoma and colon adenoma), kidney, epidermal, liver, lung, for example adenocarcinoma, small cell lung cancer and non-small cell lung carcinomas, oesophagus, gall bladder, ovary, pancreas, including exocrine pancreatic carcinoma, stomach, cervix, thyroid, prostate, or skin, for example squamous cell carcinoma; a hematopoietic tumour of lymphoid lineage, for example leukaemia, acute lymphocytic leukaemia, B-cell lymphoma, T-cell lymphoma, Hodgkin's lymphoma, non- Hodgkin's lymphoma, hairy cell lymphoma , or Burkett's lymphoma; a hematopoietic tumour of myeloid lineage, for example acute or chronic myelogenous leukemias, myelodysplastic syndrome, or promyelocytic leukaemia; thyroid follicular cancer; a tumour of mesenchymal origin, for example fibrosarcoma or habdomyosarcoma; a tumour of the central or peripheral nervous system. for example astrocytoma, neuroblastoma, glioma or schannoma; melanoma; seminoma; teratocarcinoma; osteosarcoma; xenoderoma pigmentoum; keratoctanthoma; thyroid follicular cancer; or Kaposi's sarcoma.

As mentioned above, the present invention also encompasses a method of treatment, such as a condition and/or a disease. The condition is preferably a proliferative disorder, such as cancer. The method comprises preferably the step of administering, to an individual and/or patient in need thereof, an effective amount of one or more compounds according to the invention.

The invention provides complexes as defined and disclosed in this specification, or prodrugs or solvates thereof ("active compounds"), for use in a method of treatment of the human or animal body. A method of treatment may comprise administering to such an individual a therapeutically-effective amount of the complex of the present invention, preferably in the form of a pharmaceutical composition.

The term "treatment" or "treating" is used herein in the context of treating a condition, and pertains generally to treatment and therapy, whether of a human or animal (e.g. in veterinary applications), in which some therapeutic effect is achieved, for example, the inhibition of the progress of the condition, and includes a reduction in the rate of the progress, a halt in the rate of the progress, amelioration of the condition, and cure of the condition. The condition usually is associated with suffering, from psychological and/or physical pain, with the individual being in need of a treatment. Treatment as a prophylactic measure (i.e. prophylaxis) is also included.

The complex of the invention or pharmaceutical composition comprising the active complex may be administered to an individual by any convenient route of administration, whether systemically /peripherically or at the site of desired action, including but not limited to, oral (e.g. by ingestion), topical (including e.g. transdermal, intranasal, ocular, buccal and sublingual), pulmonary (e.g. by inhalation or insufflation therapy using an aerosol, e.g. through mouth or nose), rectal, vaginal, parenteral, for example by injection, including subcutaneous, intradermal, intramuscular, intravenous, intraarterial, intracardiac, intrathecal, subcuticula, subcapsular, intraorbital, intraperitoneal, intratracheal, subarachnoid, and intrasternal, by implant of a depot (e.g. subcutaneously or intramuscularly).

The complex of the present invention may be administered alone, but is preferably presented as a pharmaceutical composition (e.g. formulation) comprising at least one active complex together with at least one or more pharmaceutically carriers, buffers, as mentioned in WO 2006/018649, p. 16-20, "formulations", which reference is expressly incorporated herein by reference.

The present invention is described more concretely by way of the following examples, which, however, are not intended to be understood as any kind of restriction of the scope of the present invention.

### Examples

### General comments

All reagents were purchased either from Aldrich or Fluka and used as received. The dimers [(arene)₂RuCl₂]₂ were prepared according to literature methods ((a) M. A. Bennett, T. N. Huang, T. W. Matheson, A. K. Smith, Inorg. Synth., 1982, 21, 74. (b) M. A. Bennett, T. W. Matheson, G. B. Robertson, A. K. Smith, P. A. Tucker, Inorg. Chem., 1980, 19, 1014. (c) M. A. Bennett, A. K. Smith, Dalton Trans., 1974, 2, 233. (d) R. A. Zelonka, M. C. Baird, Can. J. Chem., 1972, 50, 3063). Reactions were carried out under nitrogen. NMR spectra were recorded on a Bruker AMX 400 spectrometer using the residual protonated solvent as internal standard. Micro-analyses were performed by the Laboratory of Pharmaceutical Chemistry, University of Geneva (Switzerland) or by Mikroelementaranalytisches Laboratorium, ETH Zürich (Switzerland). Electrospray ionisation mass spectra were obtained in positive-ion mode with an LCQ Finnigan mass spectrometer or performed at the Department of Chemistry of the University of Fribourg (Switzerland).

### Example 1: Synthesis and characterisation of the diene precursors 1- 4

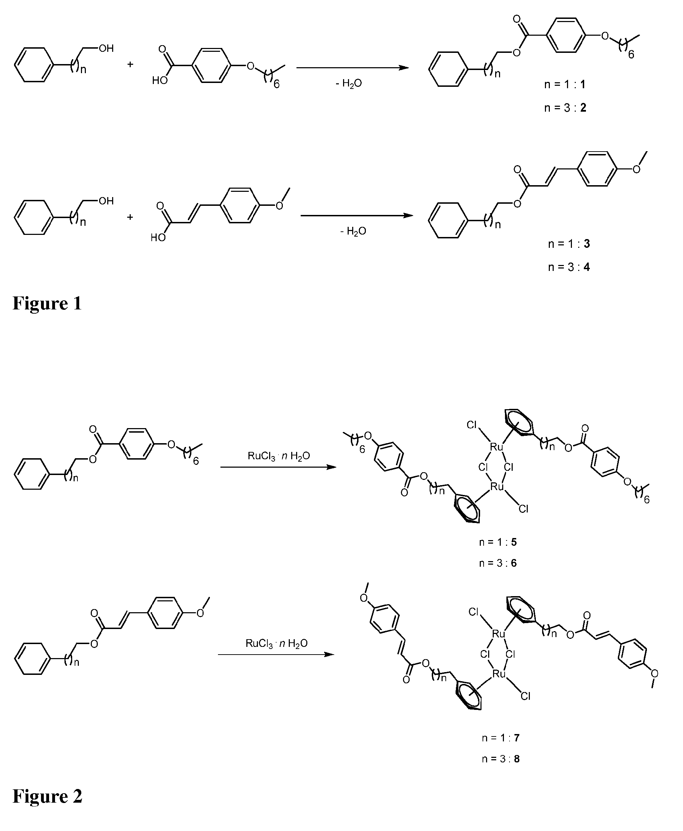

The compounds 2-(cyclohexa-1,4-dienyl)ethanol and 4-(cyclohexa-1,4-dienyl)butanol, accessible by Birch reduction of the corresponding benzene derivatives, (P. S. Engel, R. L. Allgren, W.-K. Chae, R. A. Leckonby, N. A. Marron, J. Org. Chem., 1979, 24, 4233 and F. K. Cheung, C. Lin, F. Minissi, A. L. Crivillé, M. A. Graham, D. J. Fox, M. Wills, Org. Lett., 2007, 22, 4659) react in the presence of 4-(dimethylamino)pyridine, *N,N*'-dicyclohexylcarbodiimide and 4-pyrrolidinopyridine as coupling reagents, with 4-(heptyloxy)benzoic acid to give the functionalised dienes **1** and **2,** using conditions similar to those reported previously (W. H. Ang, L. J. Parker, A. De Luca, L. Juillerat-Jeanneret, C. J. Morton, M. Lo Bello, M. W. Parker, P. J. Dyson, Angew. Chem. Int. Ed., 2009, 48, 3854). The coupling with 4-methoxycinnamic acid under the same conditions yields to the functionalised dienes **3** and **4** (**Figure 1**). The new compounds, obtained as colorless oils, were characterised by NMR spectroscopy, mass spectrometry and elemental analysis.

More specifically, to a Schlenk tube, the corresponding acid (5.2 mmol, 1.24 g for **1;** 4.3 mmol, 1.01 g for **2;** 5.2 mmol, 934 mg for **3;** 4.3 mmol, 761 mg for **4**), in the presence of the coupling reagents 4-(dimethylamino)pyridine (4 mmol, 492 mg for **1** and **3;** 3.3 mmol, 401 mg for **2** and **4**), *N,N*'-dicyclohexylcarbodiimide (8 mmol, 1.66 g for **1** and **3;** 6.6 mmol, 1.36 g for **2** and **4**) and 4-pyrrolidinopyridine (4 mmol, 597 mg for **1** and **3;** 3.3 mmol, 497 mg for **2** and **4**) were dissolved in CH₂Cl₂ (100 mL). After the addition of a solution of the diene (4 mmol, 500 mg for **1** and **3;** 3.3 mmol, 500 mg for **2** and **4**) in CH₂Cl₂ (10 mL), the reaction was stirred at 20 °C during 18 h. Then resulting mixture was filtered through celite and the solvent was removed under reduced pressure. The resulting oil was purified by column chromatography (silica gel, CH₂Cl₂/EtOH 4.8 : 0.2); compounds **1- 4** were isolated as colorless oils and dried *in vacuo.*
1. Colorless oil, yield: 1.15 g, 84%. ¹H NMR (400 MHz, CDCl₃): 0.91 (t, 3H, -C*H*₃), 1.44 (m, 8H, -CH₂-(C*H*₂)₄-CH₃), 1.80 (q, 2H, -O-CH₂-C*H*₂-(CH₂)₄-CH₃), 2.42 (t, 2H, C₆H₇-C*H*₂-CH₂-O-(CO)-Ar), 2.69 (s, 4H, C*H*₂diene), 3.99 (t, 2H, -O-C*H*₂-(CH₂)₅-CH₃), 4.38 (t, 2H, C₆H₇-CH₂-C*H*₂-O-(CO)-Ar), 5.55 (s, 1H, *CH*diene), 5.71 (t, 2H, *CH*diene), 6.89 (d, ³*J* = 6.8 Hz, 2H, *H*-Ar), 7.98 ppm (d, ³*J*= 6.8 Hz, 2H, *H*-Ar). ¹³C{¹H} NMR (100 MHz, CDCl₃): 14.18, 22.70, 26.05, 26.88, 29.13, 29.22, 29.26, 31.86, 35.42, 36.73, 60.66, 63.08, 65.23, 68.28, 114.09, 120.98, 124.17, 126.61, 131.64, 138.15, 163.07, 166.38 ppm. Mass (ESI, m/z): 365.18 [M+Na⁺]. Anal Calc. for (%): C₂₂H₃₀O₃ : C, 77.16; H, 8.83. Found: C, 77.25; H, 8.69.
2. Colorless oil, yield: 1.16 g, 95%. ¹H NMR (400 MHz, CDCl₃): 0.91 (t, 3H, -C*H*₃), 1.33 (m, 10H, -C*H*₂-(C*H*₂)₄-CH₃, C₆H₇-CH₂-C*H*₂-CH₂-CH₂-O-(CO)-Ar), 1.79 (m, 4H, C₆H₇-CH₂-CH₂-C*H*₂-CH₂-O-(CO)-Ar, -O-CH₂-C*H*₂-(CH₂)₄-CH₃), 2.05 (t, 2H, C₆H₇-C*H*₂-(CH₂)₃-O-(CO)-Ar), 2.62 (m, 2H, C*H*₂diene), 2.70 (m, 2H, C*H*₂diene), 4.01 (t, 2H, -O-C*H*₂-(CH₂)₅-CH₃), 4.31 (t, 2H, C₆H₇-(CH₂)₃-C*H*₂-O-(CO)-Ar), 5.46 (s, 1H, CHdiene), 5.72 (t, 2H, C*H*diene), 6.91 (d, ³*J* = 6.8 Hz, 2H, *H*-Ar), 8.00 ppm (d, ³*J* = 6.8 Hz, 2H, *H-*Ar). ¹³C{¹H} NMR (100 MHz, CDCl₃): 14.10, 22.62, 25.26, 25.98, 26.78, 27.87, 28.46, 28.88, 29.06, 29.15, 31.79, 37.05, 64.61, 68.21, 114.05, 118.73, 124.30, 124.32, 125.84, 128.36, 131.53, 134.53, 162.93, 166.48 ppm. Mass (ESI, m/z): 393.10 [M+Na⁺]. Anal Calc. for (%): C₂₄H₃₄O₃ : C, 77.80; H, 9.25. Found: C, 77.91; H, 9.39.
3. Colorless oil, yield: 1.02 g, 89%. ¹H NMR (400 MHz, CDCl₃): 2.37 (t, 2H, C₆H₇-C*H*₂-CH₂-O-(CO)-CH=CH-), 2.69 (m, 4H, C*H*₂diene), 3.82 (s, 3H, -O-C*H*₃), 4.29 (t, 2H, C₆H₇-CH₂-C*H*₂-O-(CO)-CH=CH-), 5.53 (s, 1H, C*H*diene), 5.71 (t, 2H, C*H*diene), 6.30 (d, ³J = 16 Hz, 1H, -C*H*=CH-), 6.91 (d, 2H, *H*-Ar), 7.47 (d, 2H, *H*-Ar), 7.64 ppm (d, ³*J* = 16 Hz, 1H, - CH=C*H*-). ¹³C{¹H} NMR (100 MHz, CDCl₃): 26.85, 36.64, 55.42, 62.78, 64.93, 114.38, 115.83, 120.88, 124.21, 126.61, 129.11, 129.76, 129.81, 144.43, 161.44, 161.48, 167.27, 167.34 ppm. Mass (ESI, m/z): 307.20 [M+Na⁺]. Anal Calc. for (%): C₁₈H₂₀O₃ : C, 77.03; H, 7.09. Found: C, 77.26; H, 7.15.
4. Colorless oil, yield: 870 mg, 84%. ¹H NMR (400 MHz, CDCl₃): 1.54 (m, 2H, C₆H₇-CH₂-C*H*₂-(CH₂)₂-O-(CO)-CH=CH-), 1.71 (m, 2H, C₆H₇-(CH₂)₂-C*H*₂-CH₂-O-(CO)-CH=CH-), 2.03 (t, 2H, C₆H₇-C*H*₂-(CH₂)₃-O-(CO)-CH=CH-), 2.61 (m, 2H, C*H*₂diene), 2.69 (m, 2H, CH₂diene), 3.84 (s, 3H, -O-C*H*₃), 4.21 (t, 2H, C₆H₇-(CH₂)₃-C*H*₂-O-(CO)-CH=CH-), 5.45 (m, 1H, C*H*diene), 5.72 (m, 2H, C*H*diene), 6.32 (d, ³*J* = 16 Hz, 1H, -C*H*=CH-), 6.91 (d, 2H, *H-*Ar), 7.49 (d, 2H, *H*-Ar), 7.65 ppm (d, ³*J*= 16 Hz, 1H, -CH=C*H*-). ¹³C{¹H} NMR (100 MHz, CDCl₃): 23.68, 26.78, 28.22, 28.88, 37.04, 53.45, 55.37, 64.39, 114.33, 115.75, 118.71, 124.32, 125.84, 127.23, 128.43, 129.71, 134.54, 144.27, 161.37, 167.42 ppm. Mass (ESI, m/z): 335.07 [M+Na⁺]. Anal Calc. for (%): C₂₀H₂₄O₃ : C, 76.89; H, 7.74. Found: C, 76.95; H, 7.62.

### Example 2: Preparation of the arene ruthenium chloro intermediates 5-8

The complexes [(C₆H₅R)RuCl₂]₂ (where R = (CH₂)₂-O-C(O)-C₆H₄-*p*-O(CH₂)₆CH₃: **5,** (CH₂)₄-O-C(O)-C₆H₄-*p*-O(CH₂)₆CH₃: **6,** (CH₂)₂-O-C(O)-CH=CH-C₆H₄-*p*-OCH₃: **7,** (CH₂)₄-O-C(O)-CH=CH-C₆H₄-*p*-OCH₃: **8),** are accessible from the reaction of the corresponding dienes **1, 2, 3** and **4** with ruthenium(III) chloride hydrate in refluxing acetone/water (5: 1), see the scheme in **Figure 2****.** Complexes **5 - 8** are air-stable brown to orange crystalline solids, which are soluble in dichloromethane and chloroform.

Specifically, To a solution of RuCl₃ · nH₂O (0.8 mmol, 209 mg for **5** and **7;** 0.6 mmol, 155 mg for **6** and **8**) in degassed acetone/water 5:1 (100 mL) a solution of the corresponding diene (4 mmol, 1.37 g for **5,** 1.14 g for 7; 3 mmol, 1.10 g for **6,** 937 mg for **8)** in degassed acetone/water 5:1 (10 mL) was added and the mixture was refluxed for 18 h. The solvent was removed under reduced pressure, and the residue was dissolved in dichloromethane (5 mL). Then the product was precipitated by addition of diethyl ether (50 mL), isolated by filtration and dried *in vacuo.*
5. Orange solid, yield: 380 mg, 93%. ¹H NMR (400 MHz, CDCl₃): 0.83 (t, 6H, -C*H*₃), 1.37 (m, 16H, -CH₂-(C*H*₂)₄-CH₃), 1.79 (q, 4H, -O-CH₂-C*H*₂-(CH₂)₄-CH₃), 3.04 (t, 4H, C₆H₅-C*H*₂-CH₂-O-(CO)-Ar), 3.98 (t, 4H, -O-C*H*₂-(CH₂)₅-CH₃), 4.54 (t, 4H, C₆H₅-CH₂-C*H*₂-O-(CO)-Ar), 5.50 (d, 4H, C*H*ₐᵣₑₙₑ), 5.66 (m, 6H, C*H*ₐᵣₑₙₑ), 6.86 (d, ³*J*=6.8 Hz, 4H, *H*-Ar), 7.86 ppm (d, ³*J*=6.8 Hz, 4H, *H*-Ar). ¹³C{¹H} NMR (100 MHz, CDCl₃): 14.19, 22.46, 22.72, 26.06, 29.13, 29.21, 31.87, 33.70, 34.25, 62.91, 68.40, 80.48, 81.37, 84.22, 97.68, 114.35, 121.73, 131.67, 163.38, 165.95 ppm. Mass (ESI, m/z): 977.20 [M-2Cl⁻+Na⁻]. Anal Calc. for (%): C₄₄H₅₆Cl₄O₃Ru₂ : C, 51.56; H, 5.51. Found: C, 51.50; H, 5.34.
6. Orange solid, yield: 290 mg, 90%. ¹H NMR (400 MHz, CDCl₃): 0.87 (t, 6H, -C*H*₃), 1.37 (m, 16H, -CH₂-(C*H*₂)₄-CH₃),1.75 (m, 12H, C₆H₅-CH₂-C*H*₂-C*H*₂-CH₂-O-(CO)-Ar, -O-CH₂-C*H*₂-(CH₂)₄-CH₃), 2.60 (t, 4H, C₆H₅-C*H*₂-(CH₂)₃-O-(CO)-Ar,), 3.97 (t, 4H, -O-C*H*₂-(CH₂)₅-CH₃), 4.26 (t, 4H, C₆H₅-(CH₂)₃-C*H*₂-O-(CO)-Ar), 5.38 (d, 4H, C*H*ₐᵣₑₙₑ), 5.60 (m, 6H, C*H*ₐᵣₑₙₑ), 6.87 (d, ³*J* = 8.8 Hz, 4H, *H*-Ar), 7.92 ppm (d, ³*J* = 8.8 Hz, 4H, *H*-Ar). ¹³C{¹H} NMR (100 MHz, CDCl₃): 14.15, 22.65, 26.00, 26.35, 28.56, 29.08, 29.17, 31.81, 33.25, 64.00, 68.29, 79.86, 80.46, 84.22, 100.99, 114.16, 122.38, 131.62, 163.06, 166.37 ppm. Mass (ESI, m/z): 1033.20 [M-2Cl⁻+Na⁺]. Anal Calc. for (%): C₄₈H₆₄Cl₄O₆Ru₂ : C, 53.33; H, 5.97. Found: C, 53.51; H, 5.94.
7. Brown solid, yield: 216 mg, 72%. ¹H NMR (400 MHz, CDCl₃): 2.85 (t, 4H, C₆H₅-C*H*₂-CH₂-O-(CO)-CH=CH-), 3.67 (s, 6H, -O-C*H*₃), 4.35 (t, 4H, C₆H₅-CH₂-C*H*₂-O-(CO)-CH=CH), 5.59 (d, 4H, *CHₐᵣₑₙₑ*), 5.77 (m, 6H, *CHₐᵣₑₙₑ*), 6.10 (d, ³*J* = 16 Hz, 2H, -C*H*=CH-), 6.74 (d, 4H, *H*-Ar), 7.31 (d, 4H, *H*-Ar), 7.44 ppm (d, ³*J*= 16 Hz, 2H, -CH=C*H*-). ¹³C{¹H} NMR (100 MHz, CDCl₃): 35.60, 55.12, 62.10, 84.34, 86.65, 88.48, 103.57, 114.10, 114.33, 126.45, 129.43, 144.93, 157.74, 161.30, 166.42, 172.13 ppm. Mass (ESI, m/z): 861.13 [M-2Cl⁻+Na⁺]. Anal Calc. for (%): C₃₆H₃₆Cl₄O₂Ru₂ : C, 47.59; H, 3.99. Found: C, 47.71; H, 4.06.
**8.** Brown solid, yield: 252 mg, 82%. ¹H NMR (400 MHz, CDCl₃): 1.73 (m, 8H, C₆H₅-CH₂-C*H*₂-C*H*₂-CH₂-O-(CO)-CH=CH-), 2.61 (t, 4H, C₆H₅-C*H*₂-(CH₂)₃-O-(CO)-CH=CH), 3.83 (s, 6H, -O-C*H*₃), 4.19 (t, 4H, C₆H₅-(CH₂)₃-C*H*₂-O-(CO)-CH=CH-), 5.39 (d, 4H, C*H*ₐᵣₑₙₑ), 5.60 (m, 6H, C*H*ₐᵣₑₙₑ), 6.28 (d, ³*J* = 16 Hz, 2H, -C*H*=CH-), 6.90 (d, 4H, *H*-Ar), 7.48 (d, 4H, *H*-Ar), 7.62 ppm (d, ³*J*= 16 Hz, 2H, -CH=C*H*-). ¹³C{¹H} NMR (100 MHz, CDCl₃): 26.41, 28.58, 33.31, 55.52, 63.85, 79.93, 80.56, 84.28, 101.08, 114.47, 115.58, 127.26, 129.91, 144.69, 161.56, 167.40 ppm. Mass (ESI, m/z): 917.07 [M-2Cl⁻+Na⁺]. Anal Calc. for (%): C₄₀H₄₄Cl₄O₂Ru₂: C, 49.80; H, 4.60. Found: C, 49.87; H, 4.66.

### Example 3: Synthesis and characterisation of the functionalised arene ruthenium thiophenolato complexes 9 -12

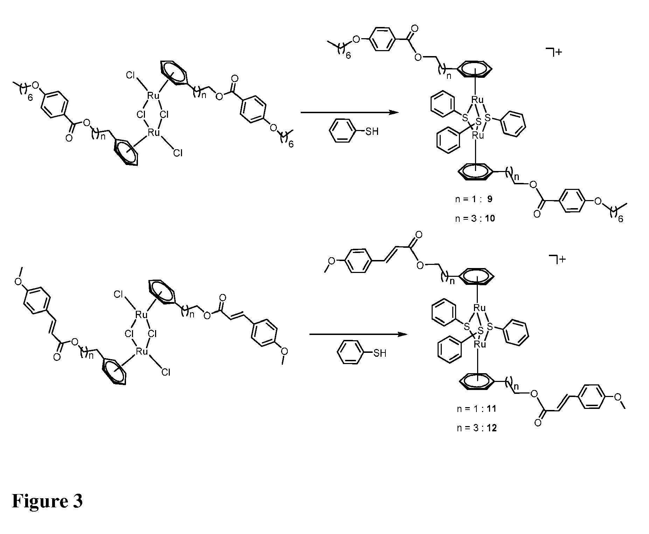

The arene ruthenium chloro intermediates **5 - 8** react with thiophenol in refluxing ethanol to give the dinuclear arene ruthenium thiophenolato complexes [(arene)₂Ru₂(SPh)₃]⁺, arene being C₆H₅R (R = (CH₂)₂-O-C(O)-C₆H₄*-p-*O(CH₂)₆CH₃: **9,** (CH₂)₄-O-C(O)-C₆H₄*-p-*O(CH₂)₆CH₃: **10,** (CH₂)₂-O-C(O)-CH=CH-C₆H₄*-p-*OCH₃: **11,** (CH₂)₄-O-C(O)-CH=CH-C₆H₄*-p-*OCH₃: **12**), according to the scheme shown in **Figure 3****.**

The chloride salts of **9 - 12** are obtained as air-stable orange to red crystalline solids which are soluble in methanol, dichloromethane and chloroform. The ¹H NMR spectra of the dinuclear arene ruthenium thiophenolato complexes [(arene)₂Ru₂(SPh)₃]⁺ **9 - 12** in CDCl₃ give rise to two signals for the three equivalent thiophenolato groups **(9:** 7.37 m, 7.88 d ppm; **10:** 7.36 m, 7.92 d ppm; **11:** 7.42 m, 7.88 d ppm; **12:** 7.41 m, 7.85 d ppm), and to the characteristic arene ligand signals. The ESI mass spectra of **9 - 12** contain molecular ion peaks at m/z 1211, 1267, 1095 and 1151, respectively.

The dinuclear dichloro complex [(arene)₂RuCl₂]₂ (0.15 mmol, 150 mg for **9,** 162 mg for **10,** 136 mg for **11,** 144 mg for **12)** was refluxed in technical grade EtOH (50 mL). As soon as the starting material was completely dissolved, a solution of thiophenol (0.9 mmol, 99 mg, 92 µL) in technical grade EtOH (5 mL) was added dropwise to the hot solution. The resulting mixture was refluxed in EtOH for 18 h. After cooling to 20 °C, the solvent was removed under reduced pressure. Tthe mixture was purified by column chromatography (silica gel, CH₂Cl₂/EtOH 5 : 1), and the compounds [**9 - 12**]Cl were isolated as air-stable orange to red crystalline solids and dried *in vacuo.*
[9]Cl. Red solid, yield: 174 mg, 93%. ¹H NMR (400 MHz, CDCl₃): 0.88 (t, 6H, -C*H*₃), 1.30 (m, 16H, -CH₂-(C*H*₂)₄-CH₃), 1.78 (q, 4H, -O-CH₂-C*H*₂-(CH₂)₄-CH₃), 2.12 (m, 4H, C₆H₅-C*H*₂-CH₂-O-(CO)-Ar), 2.36 (m, 4H, C₆H₅-C*H*₂-CH₂-O-(CO)-Ar), 3.98 (t, 4H, -O-C*H*₂-(CH₂)₅-CH₃), 4.13 (m, 4H, C₆H₅-CH₂-C*H*₂-O-(CO)-Ar), 5.62 (m, 4H, C₆H₅-CH₂-C*H*₂-O-(CO)-Ar), 5.13 (d, 2H, *CHₐᵣₑₙₑ*), 5.38 (m, 4H, *CHₐᵣₑₙₑ*), 5.62 (m, 4H, C*H*ₐᵣₑₙₑ), 6.86 (d, ³*J*= 9.2 Hz, 4H, *H*-Ar), 7.37 (m, 9H, *H*-Ar), 7.78 (d, ³*J*= 9.2 Hz, 4H, *H*-Ar), 7.88 ppm (d, 6H, *H*-Ar). ¹³C{¹H} NMR (100 MHz, CDCl₃): 14.20, 22.71, 26.05, 29.13, 29.20, 31.87, 32.18, 63.01, 68.45, 85.57, 86.03, 86.08, 86.35, 87.56, 99.80, 114.38, 121.58, 128.63, 129.59, 131.64, 132.23, 138.55, 163.42, 165.81 ppm. Mass (ESI, m/z): 1211.26 [M+H⁺]. Anal Calc. for (%): C₆₂H₇₁ClO₆Ru₂S₃ : C, 59.76; H, 5.74. Found: C, 59.81; H, 5.80.
[10]Cl. Red solid, yield: 169 mg, 91%. ¹H NMR (400 MHz, CDCl₃): 0.88 (t, 6H, -C*H*₃), 1.30 (m, 16H, -CH₂-(C*H*₂)₄-CH₃), 1.55 (m, 12H, C₆H₅-CH₂-C*H*₂-C*H*₂-CH₂-O-(CO)-Ar, -O-CH₂-C*H*₂-(CH₂)₄-CH₃), 1.80 (t, 4H, C₆H₅-C*H*₂-(CH₂)₃-O-(CO)-Ar,), 3.99 (t, 4H, -O-C*H*₂-(CH₂)₅-CH₃), 4.11 (t, 4H, C₆H₅-(CH₂)₃-C*H*₂-O-(CO)-Ar), 4.97 (d, 2H, C*H*ₐᵣₑₙₑ), 5.27 (m, 4H, C*H*ₐᵣₑₙₑ), 5.59 (m, 4H, C*H*ₐᵣₑₙₑ), 6.88 (d, ³*J*= 8Hz, 4H, *H*-Ar), 7.36 (m, 9H, *H*-Ar), 7.84 (d, ³*J* = 8 Hz, 4H, *H*-Ar), 7.92 ppm (d, 6H, *H*-Ar). ¹³C{¹H} NMR (100 MHz, CDCl₃): 14.19, 22.71, 26.06, 26.20, 28.42, 29.14, 29.23, 31.87, 32.09, 63.76, 68.41, 85.40, 86.28, 86.34, 103.70, 114.24, 122.36, 128.48, 129.47, 131.66, 132.24, 138.87, 163.20, 166.44 ppm. Mass (ESI, m/z): 1267.32 [M+H⁺]. Anal Calc. for (%): C₆₆H₇₉ClO₆Ru₂S₃ : C, 60.88; H, 6.12. Found: C, 60.81; H, 5.98.
[11]Cl. Orange solid, yield: 148 mg, 87%. ¹H NMR (400 MHz, CDCl₃): 2.76 (t, 4H, C₆H₅-C*H*₂-CH₂-O-(CO)-CH=CH-), 3.83 (s, 6H, -O-C*H*₃), 4.06 (t, 4H, C₆H₅-CH₂-C*H*₂-O-(CO)-CH=CH-), 5.12 (d, 2H, C*H*ₐᵣₑₙₑ), 5.36 (m, 4H, C*H*ₐᵣₑₙₑ), 5.61 (m, 4H, C*H*ₐᵣₑₙₑ), 6.14 (d, ³*J*= 16 Hz, 2H, -C*H*=CH-), 6.89 (d, 4H, *H*-Ar), 7.42 (m, 13H, *H*-Ar), 7.53 (d, ³*J* = 16 Hz, 2H, - CH=C*H*-), 7.88 ppm (d, 6H, *H*-Ar). ¹³C{¹H} NMR (100 MHz, CDCl₃): 32.12, 55.57, 62.73, 85.53, 86.07, 86.13, 86.37, 87.56, 99.84, 114.54, 114.61, 126.88, 128.63, 129.61, 130.06, 132.26, 138.60, 145.47, 161.79, 166.79 ppm. Mass (ESI, m/z): 1095.09 [M+H⁺]. Anal Calc. for (%): C₅₄H₅₁ClO₆Ru₂S₃ : C, 57.41; H, 4.55. Found: C, 57.52; H, 4.47.
[**12**]Cl. Orange solid, yield: 152 mg, 86%. ¹H NMR (400 MHz, CDCl₃): 1.83 (m, 8H, C₆H₅-CH₂-C*H*₂-C*H*₂-CH₂-O-(CO)-CH=CH-), 2.01 (m, 4H, C₆H₅-C*H*₂-(CH₂)₃-O-(CO)-CH=CH-), 3.82 (s, 6H, -O-C*H*₃), 4.02 (t, 4H, C₆H₅-(CH₂)₃-C*H*₂-O-(CO)-CH=CH-), 4.97 (d, 2H, C*H*ₐᵣₑₙₑ), 5.26 (m, 4H, C*H*ₐᵣₑₙₑ), 5.59 (m, 6H, C*H*ₐᵣₑₙₑ), 6.25 (d, ³*J* = 16 Hz, 2H, -C*H*=CH-), 6.89 (d, 4H, *H*-Ar), 7.41 (m, 13H, *H*-Ar), 7.60 (d, ³*J* = 16 Hz, 2H, -CH=C*H*-), 7.85 ppm (d, 6H, *H*-Ar),. ¹³C{¹H} NMR (100 MHz, CDCl₃): 15.29, 26.05, 28.26, 31.97, 55.30, 55.45, 63.42, 65.86, 84.71, 85.28, 86.18, 86.21, 103.62, 113.91, 114.41, 115.32, 127.01, 128.39, 129.24, 129.27, 132.15, 138.78, 144.65, 161.53, 167.27 ppm. Mass (ESI, m/z): 1151.15 [M+H⁺]. Anal Calc. for (%): C₅₈H₅₉ClO₆Ru₂S₃ : C, 58.74; H, 5.01. Found: C, 58.82; H, 4.85.

### Example 4: Synthesis and characterisation of the functionalised arene ruthenium hydroxythiophenolato complexes 13 - 16

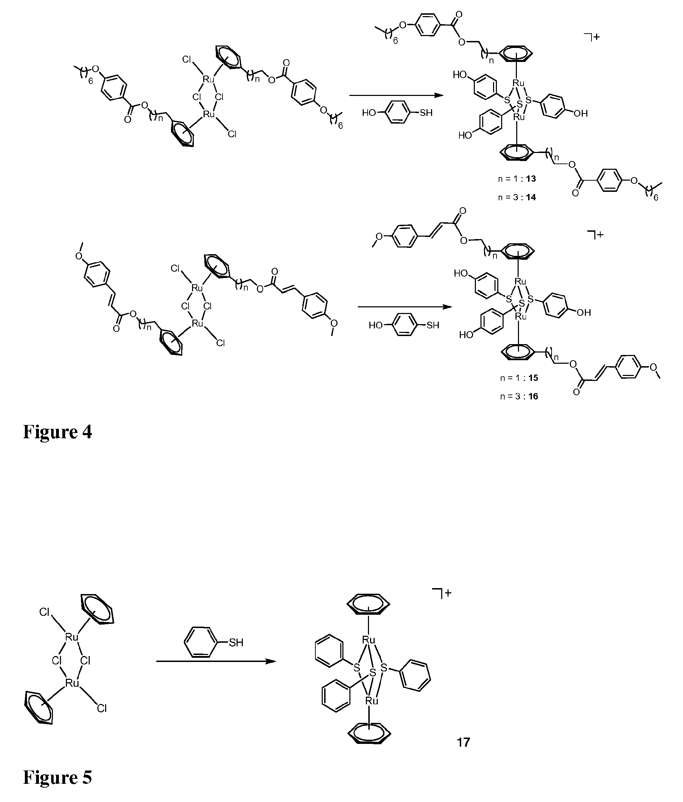

Since the anticancer activity of arene ruthenium complexes depends to some extent on their lipophilic and on their hydrophilic properties, the thiolato bridges in the [(arene)₂Ru₂(SPh)₃]⁺ complexes were modified by the introduction of a *p-*hydroxy function onto the phenyl substituent to increase water solubility. This was achieved by reacting the [(arene)RuCl₂]₂ intermediates **5 - 8** with *p-*hydroxythiophenol in a similar fashion to the method outlined above, to afford the dinuclear arene ruthenium complexes [(C₆H₅R)₂Ru₂(S*-p-*C₆H₄-OH)₃]⁺ (where R = (CH₂)₂-O-C(O)-C₆H₄*-p-*O(CH₂)₆CH₃: **13,** (CH₂)₄-O-C(O)-C₆H₄*-p-*O(CH₂)₆CH₃: **14,** (CH₂)₂-O-C(O)-CH=CH-C₆H₄*-p-*OCH₃: **15,** (CH₂)₄-O-C(O)-CH=CH-C₆H₄*-p-*OCH₃: **16),** see the reaction scheme in **Figure 4****.**

The chloride salts of **13 - 16,** which are soluble in dichloromethane, chloroform and in other polar organic solvents, are obtained as air-stable yellow to orange crystalline solids. The ¹H NMR spectra of **13 - 16** in CDCl₃ provide the two signals of the three equivalent hydroxythiophenolato groups (**13:** 7.01 d, 7.63 d ppm; **14:** 6.93 d, 7.52 d ppm; **15:** 6.90 d, 7.62 d ppm; **16:** 6.82 d, 7.47 d ppm), and the characteristic arene ligand signals (see Experimental). In the ESI mass spectra, the molecular peaks of **13 - 16** are observed at m/z 1259, 1315, 1143 and 1199, respectively.

The dinuclear dichloro complex [(arene)₂RuCl₂]₂ (0.05 mmol, 50 mg for **13,** 54 mg for **14,** 45 mg for **15,** 48 mg for **16)** was refluxed in technical grade EtOH (50 mL). As soon as the starting material was completely dissolved, a solution of *p-*hydroxythiophenol (0.3 mmol, 38 mg) in technical grade EtOH (5 mL) was added dropwise to the hot solution. The resulting mixture was refluxed in EtOH for 18 h. After cooling to 20 °C, the solvent was removed under reduced pressure. The mixture was purified by column chromatography (silica gel, CH₂Cl₂/EtOH 5 : 1) and **[13 - 16]**C1 were isolated as air-stable yellow to orange crystalline solids and dried *in vacuo.*
[**13**]C1. Orange solid, yield: 52 mg, 82%. ¹H NMR (400 MHz, CDCl₃): 0.83 (t, 6H, -C*H*₃), 1.35 (m, 16H, -CH₂-(C*H*₂)₄-CH₃), 1.71 (q, 4H, -O-CH₂-C*H*₂-(CH₂)₄-CH₃), 2.30 (m, 8H, C₆H₅-C*H*₂-CH₂-O-(CO)-Ar, C₆H₅-C*H*₂-CH₂-O-(CO)-Ar), 3.92 (t, 4H, -O-C*H*₂-(CH₂)₅-CH₃), 4.19 (m, 4H, C₆H₅-CH₂-C*H*₂-O-(CO)-Ar), 5.05 (d, 2H, C*H*ₐᵣₑₙₑ), 5.17 (m, 2H, C*H*ₐᵣₑₙₑ), 5.25 (m, 2H, C*H*ₐᵣₑₙₑ), 5.38 (m, 4H, C*H*ₐᵣₑₙₑ), 6.82 (d, 4H, *H*-Ar), 7.01 (d, 6H, *H*-Ar), 7.63 (d, 6H, *H*-Ar), 7.74 ppm (d, 4H, *H*-Ar). ¹³C{¹H} NMR (100 MHz, CDCl₃): 14.17, 22.67, 26.01, 29.17, 29.79, 31.82, 32.28, 63.47, 68.44, 84.60, 85.93, 85.97, 86.54, 100.01, 114.41, 116.95, 121.46, 127.46, 131.64, 133.25, 158.29, 163.40, 166.12 ppm. Mass (ESI, m/z): 1259.23 [M+H⁺]. Anal Calc. for (%): C₆₂H₇₁ClO₉Ru₂S₃: C, 57.55; H, 5.53. Found: C, 57.68; H, 5.59.
[**14**]C1. Orange solid, yield: 60 mg, 89%. ¹H NMR (400 MHz, CDCl₃): 0.83 (t, 6H, -C*H*₃), 1.37 (m, 16H, -CH₂-(C*H*₂)₄-CH₃), 1.52 (m, 12H, C₆H₅-CH₂-C*H*₂-C*H*₂-CH₂-O-(CO)-Ar, -O-CH₂-C*H*₂-(CH₂)₄-CH₃), 1.95 (t, 4H, C₆H₅-C*H*₂-(CH₂)₃-O-(CO)-Ar,), 3.94 (t, 4H, -O-C*H*₂-(CH₂)₅-CH₃), 4.12 (t, 4H, C₆H₅-(CH₂)₃-C*H*₂-O-(CO)-Ar), 4.85 (d, 2H, C*H*ₐᵣₑₙₑ), 5.04 (m, 4H, C*H*ₐᵣₑₙₑ), 5.29 (m, 4H, C*H*ₐᵣₑₙₑ), 6.83 (d, 4H, *H*-Ar), 6.93 (d, 6H, *H*-Ar), 7.52 (d, 6H, *H*-Ar), 7.87 ppm (d, 4H, *H*-Ar). ¹³C{¹H} NMR (100 MHz, CDCl₃): 14.09, 22.57, 25.92, 26.43, 28.30, 29.00, 29.08, 31.72, 32.13, 63.81, 68.25, 83.57, 84.70, 86.05, 86.10, 103.81, 114.09, 116.74, 122.19, 127.20, 131.55, 132.93, 158.53, 163.03, 166.36 ppm. Mass (ESI, m/z): 1315.30 [M+H⁺]. Anal Calc. for (%): C₆₆H₇₉ClO₉Ru₂S₃ : C, 58.71; H, 5.90. Found: C, 58.83; H, 5.84.
[**15**]C1. Yellow solid, yield: 49 mg, 83%. ¹H NMR (400 MHz, CDCl₃): 2.29 (m, 4H, C₆H₅-C*H*₂-CH₂-O-(CO)-CH=CH-), 3.79 (s, 6H, -O-C*H*₃), 4.09 (t, 4H, C₆H₅-CH₂-C*H*₂-O-(CO)-CH=CH-), 5.02 (d, 2H, C*Hₐᵣₑₙₑ),* 5.21 (m, 4H, C*Hₐᵣₑₙₑ),* 5.40 (m, 4H, C*H*ₐᵣₑₙₑ), 6.13 (d, ³*J* = 16 Hz, 2H, -C*H*=CH-), 6.90 (d, 6H, *H*-Ar), 7.42 (m, 8H, *H*-Ar), 7.48 (d, ³*J* = 16 Hz, 2H, - CH=C*H*-), 7.62 ppm (d, 6H, *H*-Ar). 13C{¹H} NMR (100 MHz, CDCl₃): 32.13, 55.47, 63.03, 85.81, 85.95, 96.06, 86.46, 99.86, 114.46, 116.65, 126.77, 127.52, 129.25, 130.03, 133.23, 145.58, 158.11, 161.73, 167.13 ppm. Mass (ESI, m/z): 1143.09 [M+H⁺]. Anal Calc. for (%): C₅₄H₅₁ClO₉Ru₂S₃ : C, 55.07; H, 4.36. Found: C, 55.16; H, 4.44.
[16]Cl. Yellow solid, yield: 54 mg, 88%. ¹H NMR (400 MHz, CDCl₃): 1.43 (m, 8H, C₆H₅-CH₂-C*H*₂-C*H*₂-CH₂-O-(CO)-CH=CH-), 1.89 (m, 4H, C₆H₅-C*H*₂-(CH₂)₃-O-(CO)-CH=CH-), 3.71 (s, 6H, -O-C*H*₃), 3.96 (t, 4H, C₆H₅-(CH₂)₃-C*H*₂-O-(CO)-CH=CH-), 4.78 (d, 2H, C*H*ₐᵣₑₙₑ), 4.98 (m, 4H, C*H*ₐᵣₑₙₑ), 5.24 (m, 6H, C*H*ₐᵣₑₙₑ), 6.15 (d, ³*J* = 16 Hz, 2H, -C*H*=CH-), 6.77 (d, 4H, *H*-Ar), 6.82 (d, 6H, *H*-Ar), 7.34 (d, 4H, *H*-Ar), 7.47 (d, 6H, *H*-Ar), 7.52 ppm (d, ³*J*= 16 Hz, 2H, -CH=C*H*-). ¹³C{¹H} NMR (100 MHz, CDCl₃): 15.26, 26.12, 28.12, 31.92, 55.27, 63.32, 77.37, 83.40, 84.51, 84.84, 85.98, 114.23, 115.17, 116.44, 129.61, 132.84, 144.43, 158.32, 161.32, 167.03 ppm. Mass (ESI, m/z): 1199.14 [M+H⁺]. Anal Calc. for (%): C₅₈H₅₉ClO₉Ru₂S₃: C, 56.46; H, 4.82. Found: C, 56.51; H, 4.85.

### Example 5: Synthesis and characterisation of the unfunctionalised arene ruthenium thiophenolato complexes 17 - 19

In order to study the influence of the lipophilic chains in the arene ligands on the biological activity, we also synthesised the unfunctionalised cation [(C₆H₆)₂Ru₂(SPh)₃]⁺. Complex **17** was prepared from the reaction of thiophenol with [(C₆H₆)RuCl₂]₂ in refluxing ethanol (scheme in **Figure 5**).

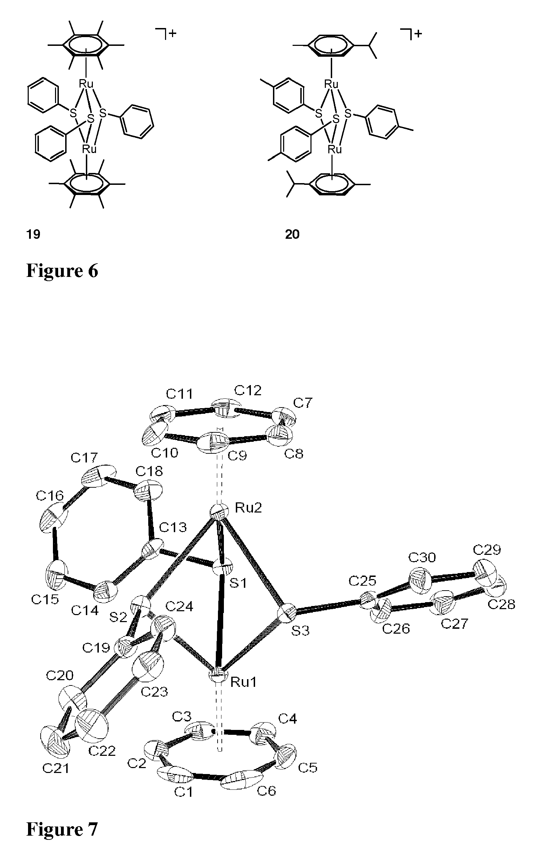

The known *p-*cymene and hexamethylbenzene analogs [(*p*-¹PrC₆H₄Me)₂Ru₂(SPh)₃]⁺ (**18**) (K. Mashima et al., Chem. Lett., 1992, 1795), [(C₆Me₆)Ru₂(SPh)₃]⁺ (**19**) (H. T. Schacht et al., Inorg. Chem., 1992, 31, 1728) (see **Figure 6**) and [(*p*-ⁱPrC₆H₄Me)₂Ru₂(S*-p-*C₆H₄Me)₃]⁺ (**20**) (F. Chérioux et al., Chem. Eur. J., 2002, 19, 4377) were also synthesised according to this method which gives much better yields than the reported procedures. All the complexes were isolated as the chloride salts.

Compound [**17**]C1, an air-stable yellow solid soluble in water and in organic solvents, gives rise to two signals for the three equivalent thiophenolato groups (7.37 m and 7.83 d ppm) and to a singlet for the two equivalent benzene ligands (5.44 ppm) in the ¹H NMR spectrum. The ESI mass spectrum shows the molecular peak at m/z 686.94. The spectroscopic data for the analogues [**18**]C1, [**19**]C1 and [**20**]C1 is in excellent agreement with that reported previously Mashima et al., Schacht et al. Chérioux et al., cited above).

The molecular structure of **17** was established by single-crystal X-ray diffraction analysis of the PF₆⁻ salt. The unit cell contains two independent cations (molecules A and B), the bond distances and angles being similar. An ORTEP drawing with the atom labelling scheme for molecule A of cation **17** is shown in **Figure 7** and selected bond lengths and angles are given in **Table 1** below. The structure contains a trigonal bipyramidal Ru₂S₃ framework, in which each ruthenium atom adopts a pseudo-octahedral geometry due to the three sulfur atoms and the benzene ligand that formally occupies three coordination sites.

**Table 1 Selected bond lengths (Å) and angles (°) for cation 17.**

| | Molecule A | Molecule B |
|---|---|---|
| *Interatomic distances* | | |
| Ru-S | 2.3764(12) | 2.3823(13) |
| | 2.3858(12) | 2.3828(13) |
| | 2.4014(12) | 2.3866(14) |
| | 2.4019(13) | 2.3961(13) |
| | 2.4112(12) | 2.4043(13) |
| | 2.4239(12) | 2.4119(14) |
| S-Cₚₕ | 1.793(5) | 1.792(5) |
| | 1.795(5) | 1.795(5) |
| | 1.798(5) | 1.796(6) |
| Ru-centroid | 1.704 | 1.695 |
| | 1.705 | 1.698 |
| | | |
| *Angles* | | |
| Ru-S-Ru | 88.17(4) | 88.58(4) |
| | 88.48(4) | 88.67(4) |
| | 89.67(4) | 88.96(4) |

The Ru-S bond distances in the cation **17** range from 2.3764(12) to 2.4239(12) Å and the Ru-S-Ru angles range from 88.17(4) to 89.67(4)°, similar to those found in the known *p-*cymene and hexamethylbenzene derivatives [(*p-*¹PrC₆H₄Me)₂Ru₂(SPh)₃]⁺ (**18**) and [(C₆Me₆)₂Ru₂(SPh)₃]⁺ (**19**), but slightly longer than in a thiopyrone complex (W. Kandioller, et al., Organometallics, 2009, 28, 4249). In accordance with the electron count, the Ru-Ru distances (3.3576(5) and 3.3481(6) Å) are clearly outside of the range for a metal-metal single bond (2.28 - 2.95 Å) (W. H. Ang, et al., Angew. Chem. Int. Ed., 2009, 48, 3854).

The three phenyl groups are not in the plane formed by the three sulfur atoms. The difference between the two independent molecules resides in the relative tilt of the phenyl groups with respect to this plane; in molecule A two phenyl groups are tilted to the right and the third phenyl group is tilted to the left whereas the tilts are opposite in molecule B.

The dinuclear dichloro complex [(arene)₂RuCl₂]₂ (0.20 mmol, 100 mg for **17**; 122 mg for **18**; 133 mg for **19**) was refluxed in technical grade EtOH (50 mL). As soon as the starting material was dissolved, a solution of thiophenol (1.2 mmol, 132 mg, 123 µL) in technical grade EtOH (5 mL) was added dropwise to the hot solution. The resulting mixture was refluxed in EtOH for 18 h. After cooling to 20 °C, the solvent was removed under reduced pressure. The mixture was purified by column chromatography (silica gel, CH₂Cl₂/EtOH 5:1) and [**17-19**]Cl were isolated as air-stable yellow to red crystalline solids and dried *in vacuo.*
[17]Cl. Yellow solid, yield: 129 mg, 88%. ¹H NMR (400 MHz, CDCl₃): 5.44 (s, 12H, *H*-Ar), 7.37 (m, 9H, *H*-Ar), 7.83 ppm (d, 6H, *H*-Ar). ¹³C{¹H} NMR (100 MHz, CDCl₃): 86.55, 128.32, 129.61, 132.05, 139.22 ppm. Mass (ESI, m/z): 686.94 [M+H⁺]. Anal Calc. for (%): C₃₀H₂₇ClRu₂S₃ : C, 49.95; H, 3.77. Found: C, 49.85; H, 3.70.
[**18**]C1. Orange solid, yield: 155 mg, 93%. ¹H NMR (400 MHz, CDCl₃): 0.78 (d, ³*J* = 8 Hz, 12H, (C*H*₃)₂CH), 1.57 (s, 6H, C*H*₃), 1.86 (sept, ³*J* = 7.2 Hz, 2H, (CH₃)₂C*H*), 5.05 (d, ³*J* = 8 Hz, 2H, *H*-Ar), 5.08 (d, ³*J* = 8 Hz, 2H, *H*-Ar), 5.19 (d, 2H, *H*-Ar), 5.36 (d, 2H, *H*-Ar), 7.34 (m, 9H, *H*-Ar), 7.84 ppm (d, 6H, *H*-Ar). ¹³C{¹H} NMR (100 MHz, CDCl₃): 17.71, 21.95, 22.53, 30.58, 83.65, 84.76, 85.03, 85.37, 99.97, 107.39, 128.45, 129.19, 132.57, 137.81 ppm. Mass (ESI, m/z): 799.07 [M+H⁺]. Anal Calc. for (%): C₃₈H₄₃ClRu₂S₃ : C, 54.76; H, 5.20. Found: C, 54.69; H, 5.32.
[**19**]C1. Red solid, yield: 162 mg, 91%. ¹H NMR (400 MHz, CDCl₃): 2.02 (s, 36H, C*H*₃), 7.36 (m, 9H, *H*-Ar), 7.87 ppm (d, 6H, *H*-Ar). ¹³C{¹H} NMR (100 MHz, CDCl₃): 14.99, 97.82, 128.42, 129.53, 132.15, 138.32 ppm. Mass (ESI, m/z): 855.13 [M+H⁺]. Anal Calc. for (%): C₄₂H₅₁ClRu₂S₃: C, 56.70; H, 5.78. Found: C, 56.65; H, 5.70.

### Example 6: Biological activity towards human ovarian cancer cells

### 1. Cell Culture and Inhibition of Cell Growth

Human A2780 and A2780cisR ovarian carcinoma cells were obtained from the European Centre of Cell Cultures (ECACC, Salisbury, UK) and maintained in culture as described by the provider. The cells were routinely grown in RPMI 1640 medium with GlutaMAX^{™} containing 5% foetal calf serum (FCS) and antibiotics (penicillin and streptomycin) at 37°C and 5% CO₂.

The antiproliferative activity of complexes **9 - 20** was evaluated against the human ovarian A2780 cancer cell line and its cisplatin-resistant derivative A2780cisR using the MTT assay, which measures mitochondrial dehydrogenase activity as an indication of cell viability. For the evaluation of growth inhibition tests, the cells were seeded in 96-well plates (25x10³ cells per well) and grown for 24 h in complete medium. Complexes were dissolved in DMSO and added to the required concentration to the cell culture for 72 h incubation. Solutions of the compounds were applied by diluting a freshly prepared stock solution of the corresponding compound in aqueous RPMI medium with GlutaMAX^{™} (20mM). Following drug exposure, 3-(4,5-dimethylthiazol-2-yl)-2,5-diphenyltetrazolium bromide (MTT) was added to the cells at a final concentration of 0.25 mg/mL and incubated for 2 h, then the culture medium was aspirated and the violet formazan (artificial chromogenic precipitate of the reduction of tetrazolium salts by dehydrogenases and reductases) dissolved in DMSO. The optical density of each well (96-well plates) was quantified three times in triplicates at 540 nm using a multiwall plate reader (iEMS Reader MF, Labsystems, US), and the percentage of surviving cells was calculated from the ratio of absorbance of treated to untreated cells. The IC₅₀ values for the inhibition of cell growth were determined by fitting the plot of the logarithmic percentage of surviving cells against the logarithm of drug concentration using a linear regression function. The median value and the median absolute deviation were obtained from the Excel™ software (Microsoft™) and those values are reported in **Table 2.**

### 2. Results and Discussion

The IC₅₀ values of **9 - 20**, corresponding to inhibition of cancer cell growth at the 50% level, are listed in Table 2 below.

**Table 2 IC₅₀ values of complexes 9 - 20 towards A2780 and A2780cisR human ovarian cancer cells.**

| Compound | IC₅₀ (µM) | IC₅₀ (µM) |
|---|---|---|
| | A2780 | A2780cisR |
| [**9**]Cl | 2.2 | 2.7 |
| [**10**]Cl | 4.9 | 5.6 |
| [**11**]Cl | 0.82 | 1.36 |
| [**12**]Cl | 0.49 | 0.56 |
| [**13**]Cl | 127 | 132 |
| [**14**]Cl | 130 | 126 |
| [**15**]Cl | 113 | 104 |
| [**16**]Cl | 13.6 | 27.2 |
| [**17**]Cl | 0.38 | 0.48 |
| [**18**]Cl | 0.24 | 0.25 |
| [**19**]Cl | 0.4 | 1.2 |
| [**20**]Cl | 0.13 | 0.08 |

The complexes tested show a broad range of cytotoxicities, depending on both the thiophenyl and arene substituents, but with comparable effects on both the cisplatin sensitive and resistant cell lines. More precisely, two general tendencies can be appreciated from the data. First, the arene ruthenium thiophenato complexes (**9** to **12**) are systematically more cytotoxic than their hydroxyl-thiophenato analogues (**13** to **16**). Second, the arene moiety have an effect, as shown by the diversity of toxicities observed for compounds **9, 10, 17, 18, 19** and **20**, although the IC₅₀ values obtained cannot be precisely correlated to the lipophilicity or the size of the substituents. It is worth noting that the sub-micromolar cytotoxicites observed for some of these compounds place them amongst the most cytotoxic arene ruthenium compounds reported, even based on the fact that two ruthenium centers are present.

The stability of the most active complex [(*p-*¹PrC₆H₄Me)₂Ru₂(S*-p-*C₆H₄Me)₃]⁺ (**20**) was assessed using ¹H NMR spectroscopy over 12 hours at 37°C in DMSO-d₆, in D₂O/DMSO-d₆ (95:5), and in DMSO-d₆ in the presence of two drops of RPMI 1640 medium with GlutaMAX^{™} containing 5% foetal calf serum and penicillin and streptomycin antibiotics to mimic pseudo-physiological conditions. The ¹H NMR signals of **20** did not change at all during 12 hour period which indicates that it is stable under these conditions.

In conclusion, a series of highly cytotoxic cationic diruthenium complexes of general formula [(arene)₂Ru₂(SPh)₃]⁺ have been prepared. Without wishing to be bound by theory, the high in vitro anticancer activity of these complexes could be ascribed, at least in part, to the presence of the thiophenolato groups. Further work is required to delineate the mode of action of these compounds and to develop further derivatives in order to understand the role of the bridging thiolato, in particular thiophenolato, selenolato, alkoxo and/or amido ligands.

## Claims

1. A cationic complex for use as a medicament, the complex comprising the structure of formula (I): 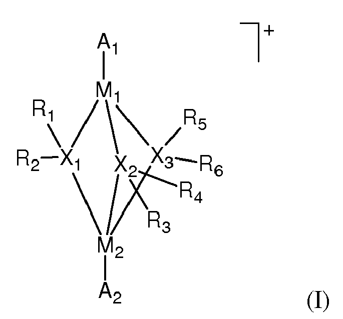
wherein M₁ and M₂ are metal atoms selected independently from Ru, Os and Fe;
wherein A₁ and A₂ are arenes bound as η-6 ligands to the metal atoms M₁ and M₂, respectively, said arenes being selected from substituted or unsubstituted benzene and substituted or unsubstituted polycycles of up to three rings and comprising at least one benzene ring;
wherein said substituents, if present, may be selected from C1-C20 hydrocarbons optionally comprising 0-15 heteroatoms, halogens, hydroxyl, carboxylic acid group, carbonyl, nitro and amino;
wherein X₁, X₂, and X₃ are, independently, selected from S, Se, O, and N;
wherein, if any one of X₁, X₂, and X₃ is other than N, R₂, R₄, and R₆, respectively, is/are absent;
wherein R₁, R₂, R₃, R₄, R₅ and R₆, in as far as present, are selected, independently, from H, and from C1-C30 hydrocarbons optionally comprising 0-20 heteroatoms, said hydrocarbons being selected from:
substituted or unsubstituted aryls;
substituted or unsubstituted aliphatic substituents; and
substituents derived independently from a natural compound and/or a bioactive compound;
wherein substituents of said aryl and aliphatic substituent, if present, may be selected independently from C1-C20 hydrocarbons comprising 0-15 heteroatoms, halogens, hydroxy, carboxylic acid group, nitro and amino, and wherein two substituents provided on different carbons of said aryl or aliphatic substituent may be connected so as to form one or more ring fused to said aryl or aliphatic substituent.

2. The complex of claim 1, comprising a structure of formula (II): 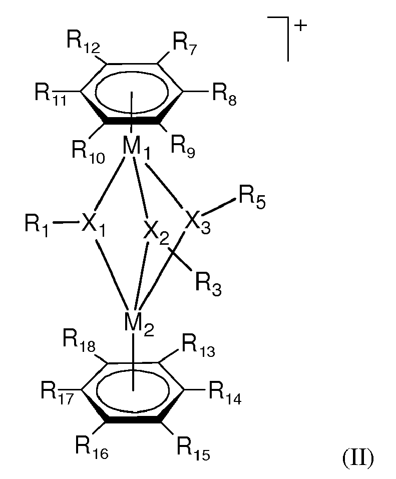
wherein M₁, M₂, R₁, R₃ and R₅, are as defined in claim 1;
wherein X₁, X₂ and X₃ are selected from S, Se and O;
wherein substituents R₇, R₈, R₉, R₁₀, R₁₁, R₁₂, R₁₃, R₁₄, R₁₅, R₁₆, R₁₇, and R₁₈ are selected, independently, from:
C4-C20 hydrocarbons optionally comprising 0-15 heteroatoms, with the proviso that said C4-C20 hydrocarbon optionally comprising 0-15 heteroatoms comprises at least one aromatic ring or ring system and/or comprises at least one bioactive moiety,
and further from:
H, halogens, hydroxyl, carboxylic acid group, nitro and amino, C1-C10 alkyls, C2-C10 alkenyls, C2-C10 alkynyls, C4-C10 aryl, C1-C10 alkoxyl, C2-C10 alkenoxyl, C2-C10 alkynoxyl, C4-C10 aryloxyl, wherein said alkyl, alkoxyl, alkenyl, alkenoxyl, alkynyl, alkynoxyl, may be totally or partially halogenated and may be linear or branched, wherein said aryl and/or an aryl moiety of said aryloxyl, if it is a C4 or a C5 aryl and/or aryloxyl, respectively, it comprises at least on heteroatom, wherein said aryl and aryloxyl may be further substituted, besides with halogen, with C1-C4 alkyl, and wherein substituents on neighbouring carbons of the benzene ring may be linked so as to form one or more rings fused to said benzene ring.

3. The complex of any one of the preceding claims, comprising a structure of formula (III): 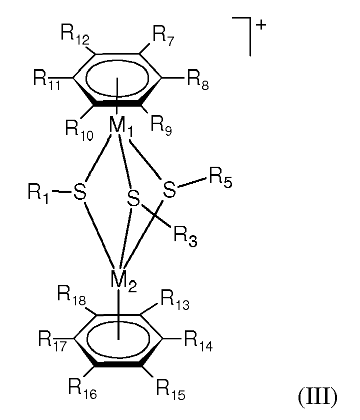 wherein:
M₁ M₂, R₁, R₃, R₅, R₇, R₈, R₉, R₁₀, R₁₁, R₁₂, R₁₃, R₁₄, R₁₅, R₁₆, R₁₇, and R₁₈ are independently as defined in claim 2.

4. The complex of any one of the preceding claims, wherein, in said R₁, R₂, R₃, R₄, R₅ and R₆, in as far as present,
said aryl is a substituted or unsubstituted phenyl;
said aliphatic substituent is selected from C1-C10 alkyl, C2-C10 alkenyl, C2-C10 alkynyl,
wherein said alkyl, alkenyl and alkynyl may be substituted and may be linear and, if it comprises at least three carbons, also branched and/or cyclic and, wherein, in said alkyl, alkenyl ad alkynyl, one or more carbons may be replaced by heteroatoms;
wherein substituents of said phenyl, alkyl, alkenyl, and alkynyl, if present, may be independently selected from C1-C20 hydrocarbons optionally comprising 0-15 heteroatoms.

5. The complex of claim 4, wherein optional substituents of said phenyl, alkyl, alkenyl, and alkynyl, if present, are selected from:
halogen;
-R₃₀;
-O-R₃₀;
-O-R₃₅;
wherein R₃₀ is independently selected from C1-C8 alkyls, C2-C8 alkenyls, and C2-C8 alkynyls, which, independently, may be linear, and if comprising more than three carbons, branched or cyclic, and which may be totally or partially halogenated, and from substituents of formula (V) below: 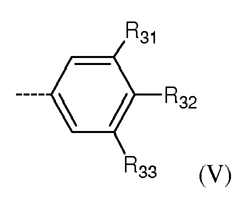 wherein R₃₅ is derived from a C3-C20 hydrocarbon compound comprising 1 to 15 heteroatoms, said compound being selected from natural compounds and bioactive compounds comprising at least one carboxylic acid group forming, in said substituent R₃₅, an ester bond with the oxygen atom of said -O-R₃₅ substituent;
wherein R₃₁, R₃₂, R₃₃ are, independently, selected from:
H;
halogen;
C1-C8 alkyl, C2-C8 alkenyl, C2-C8 alkynyl, which may linear or, if comprising three or more carbons, branched and cyclic, wherein said alkyls, alkenyls and alkynyl may be totally or partially halogenated; and,
substituents -O-R₃₅.

6. The complex of any one of the preceding claims, wherein, if said substituents R₁, R₂, R₃, R₄, R₅ and R₆, in as far as present, are derived from a natural compound and/or bioactive compound, said compound comprises at least one ligand group selected from HS-, HSe-, HO-, a primary amino group, a secondary amino group, or wherein said natural or bioactive compound is covalently connected to a linker moiety comprising one of the aforementioned ligand groups, the linker moiety comprising, said ligand group included, 1-10 carbons and 1-5 heteroatoms, and wherein, in said substituent derived from a natural compound and/or a bioactive compound, the sulphur, selenium, oxygen or nitrogen group of said HS-, HSe-, HO-, primary amino, or secondary amino ligand group, respectively, constitutes one or more of X₁, X₂, and X₃ in the complex.

7. The complex of any one of the preceding claims, comprising a structure of formula (IV): 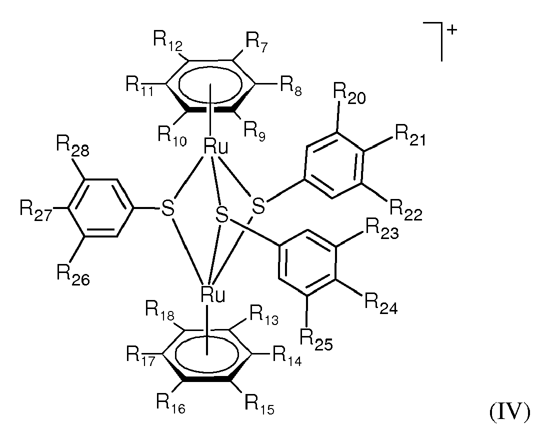 wherein:
R₇, R₈, R₉, R₁₀, R₁₁, R₁₂, R₁₃, R₁₄, R₁₅, R₁₆, R₁₇, and R₁₈ are independently as defined in claim 2;
R₂₀, R₂₁, R₂₂ R₂₃, R₂₄, R₂₅, R₂₆, R₂₇, R₂₈ are independently selected from H, halogen, -R₃₀, -O-R₃₀, -O-R₃₅, wherein -R₃₀ and -O-R₃₅ are defined as in claim 5.

8. The complex of claim 5 or 7, wherein R₃₅ is derived from any one compound selected from isonicotinic acid, ethacrynic acid, folic acid, biotin, pyridoxol-5-phosphate hydrate, wherein any carboxylic acid group of said compound forms an ester bond with the oxygen atom of the -O-R₃₅ substituent.

9. The complex of claim 7, wherein R₂₀, R₂₁, R₂₂, R₂₃, R₂₄, R₂₅, R₂₆, R₂₇, R₂₈, in as far as present, are selected from H, C1-C4 alkyl, wherein said alkyl, if it has 3 or 4 carbons, may be branched.

10. The complex of any one of claims 2 to 9, wherein:
R₇, R₈, R₉, R₁₀, R₁₁, R₁₂, R₁₃, R₁₄, R₁₅, R₁₆, R₁₇, and R₁₈ are independently selected from: H, halogens, hydroxyl, carboxylic acid group, nitro and amino, C1-C8 alkyls, C2-C8 alkenyls, C2-C8 alkynyls, C4-C8 aryl, C1-C8 alkoxyl, C2-C8 alkenoxyl, C2-C8 alkynoxyl, C4-C10 aryloxyl, all of which may be totally or partially halogenated and wherein said alkyl, alkoxyl, alkenyl, alkenoxyl, alkynyl, alkynoxyl, and aryloxyl may be linear or branched, wherein, if said aryl and/or an aryl moiety of said aryloxyl, if it is a C4 or a C5 aryl and/or aryloxyl, respectively, it comprises at least on heteroatom, and wherein substituents on neighbouring carbons of the benzene ring may be linked so as to form one or more rings fused to said benzene ring.

11. The complex of any one of claims 2 to 9, wherein R₇, R₈, R₉, R₁₀, R₁₁, R₁₂, R₁₃, R₁₄, R₁₅, R₁₆, R₁₇, and R₁₈ are independently selected from:
H, C1-C5 alkyls, C2-C5 alkenyls, and C2-C5 alkynyls, wherein said alkyl, alkenyl and alkynyl may be linear or branched, and from:
- (CH₂)ₙ-O-C(O)-C₆H₄*-p-*O-(CH₂)ₘCH₃,
- (CH₂)ₙ-O-C(O)-CH=CH-C₆H₄*-p-*O-CH₃,
wherein m is, independently, an integer of 1-6 and n being, independently, 1, 2, 3 or 4.

12. The complex of any one of the preceding claims, which is provided together with a pharmaceutically acceptable anionic species.

13. The complex of any one of the preceding claims, which has the formula [(arene)₂Ru₂(SY)₃]⁺, wherein
arene is selected from C₆H₆ (benzene), C₆H₅Me (methylbenzene), C₆Me₆
(hexamethylbenzene), *p-*ⁱPrC₆H₄Me (*p-*cymene), C₉H₁₀ (indane),
C₆H₆-(CH₂)ₙ-O-C(O)-C₆H₄*-p-*O-(CH₂)ₘCH₃,
C₆H₆-(CH₂)ₙ-O-C(O)-CH=CH-C₆H₄*-p-*O-CH₃, with m being an integer of 1-6 and n being 1, 2, 3 or 4; and,
Y is selected from phenyl and C1-C3 alkylphenyl, in particular *p-*C1-C3 alkylphenyl.

14. The complex of any one of the preceding claims, which comprises at least one binuclear ruthenium cation selected from the group of:
[(C₆H₆)₂Ru₂(SPh)₃]⁺,
[(C₆H₆)Ru₂(S*-p-*C₆H₄Me)₃]⁺,
[(C₆H₅Me)₂Ru₂(S-Ph)₃]⁺,
[(C₆H₅Me)₂Ru₂(S*-p-*C₆H₄Me)₃]⁺,
[(C₆Me₆)₂Ru₂(SPh)₃]⁺,
[(C₆Me₆)₂Ru₂(S*-p-*C₆H₄Me)₃]⁺,
[(*p-*ⁱPrC₆H₄Me)₂Ru₂(SPh)₃]⁺,
[(*p-*ⁱPrC₆H₄Me)₂Ru₂(S*-p-*C₆H₄Me)₃]⁺,
[(C₆H₆-(CH₂)₂-O-C(O)-C₆H₄*-p-*O-(CH₂)₆CH₃)₂Ru₂(SPh)₃]⁺,
[(C₆H₆-(CH₂)₂-O-C(O)-C₆H₄*-p-*O-(CH₂)₆CH₃)₂Ru₂(S*-p-*C₆H₄Me)₃]⁺, [(C₆H₆-(CH₂)₄-O-C(O)-C₆H₄*-p-*O-(CH₂)₆CH₃)₂Ru₂(SPh)₃]⁺,
[(C₆H₆-(CH₂)4-O-C(O)-C₆H₄*-p-*O-(CH₂)₆CH₃)₂Ru₂(S*-p-*C₆H₄Me)₃]⁺, [*trans*-(C₆H₆-(CH₂)₂-O-C(O)-CH=CH-C₆H₄*-p-*O-CH₃)₂Ru₂(SPh)₃]⁺,
[*trans*-(C₆H₆-(CH₂)₂-O-C(O)-CH=CH-C₆H₄*-p-*O-CH₃)₂Ru₂(S*-p-*C₆H₄Me)₃]⁺,
[*trans*-(C₆H₆-(CH₂)₄-O-C(O)-CH=CH-C₆H₄*-p-*O-CH₃)₂Ru₂(SPh)₃]⁺ ,
[*trans*-(C₆H₆-(CH₂)₄-O-C(O)-CH=CH-C₆H₄*-p-*O-CH₃)₂Ru₂(S*-p-*C₆H₄Me)₃]⁺.

15. The complex of any one of the preceding claims, for use in the treatment of hyperproliferative disorders.
